# EUROPEAN PATENT APPLICATION

(11) **EP 2 036 556 A1**
(43) Date of publication of application: **18.03.2009**
(21) Application number: 07017891.8
(22) Date of filing: 12.09.2007
(51) Int. Cl.: A61K 31/451, A61K 31/4523, A61K 31/4545, A61P 31/12, A61P 31/18, A61P 33/02, A61P 35/00, A61P 35/02, C07D 211/44

(54) **Piperidone derivatives and medical uses thereof**

(71) Applicant: Heinrich-Pette-Institut, 20251 Hamburg (DE); Julius-Maximilians-Universität Würzburg, 97970 Würzburg (DE)
(72) Inventor: Hauber, Joachim, Prof. Dr., 22527 Hamburg (DE); Hauber, Ilona, 22527 Hamburg (DE); Holzgrabe, Ulrike, Prof. Dr., 97246 Eibelstadt (DE); Klöckner, Jessica, 97074 Würzburg (DE)
(74) Representative: Weber, Martin

(57) **Abstract**

The present invention relates to compositions comprising 4-Oxo-piperidine-carboxylates and derivatives thereof, for example derivatives substituted with phenyl, nitrophenyl or benzyl groups. It also teaches the medical use of these and related compounds for treatment of retroviral diseases, in particular, HIV and HTLV, proliferative diseases, in particular CML and Trypanosomiasis.

## Description

The present invention relates to compositions comprising 4-oxo-piperidine-carboxylates and derivatives thereof, for example derivatives substituted with phenyl or nitrophenyl in 2- and 6-position and benzyl or allyl groups attached to the N. It also teaches the medical use of these and related compounds for treatment of retroviral diseases, in particular, HIV and HTLV, proliferative diseases, in particular CML and trypanosomiasis.

Retroviral infections such as infections by the Human immunodeficiency virus (HIV) or Human T-cell lymphotropic virus (HTLV) belong to the most important and most widespread human diseases.

With regard to AIDS, an estimated 39.5 million people are living with HIV, the retrovirus causing AIDS. Recent data show that even in 2006, there were about 4.3 million new infections, with some areas in the world where the infection rates have risen by more than 50% since 2004. Further, in 2006, about 2.9 million people died of AIDS-related illnesses according to the AIDS Epidemic Update 2006, published by the WHO.

The primary goals of antiretroviral therapy applied are a reduction of HIV-related morbidity and mortality, an improvement of quality of life, a restoration and preservation of immunologic function, and maximal and durable suppression of viral load. Today antiretroviral therapy, more precisely, treatment regimens against HIV, primarily rely on virus enzyme inhibitors and molecules that inhibit virus-cell fusion. In this regard, there are currently four classes of anti-HIV medicines available that are used in the treatment of AIDS. These drug classes target specific steps in the HIV replication process.

Fusion inhibitors (FI) are the first class of active agents, which work outside of host cells in order to prevent HIV from fusing with, entering and infecting these cells. A related approach is to prevent binding of HIV to the target cell via CD4 receptors and co-receptors called CCR5 or CXCR4. The three other classes of active agents work inside the cell. Nucleoside reverse transcriptase inhibitors, non-nucleoside reverse transcriptase inhibitors and protease inhibitors are used to prevent the replication of the virus inside host cells after they have been infected with HIV.

One type of combination therapy currently used involving the use of more than one active agent is the Highly Active Antiretroviral Therapy (HAART) targeting the viral reverse transcriptase, protease and fusion. The application of this therapy has resulted in transforming HIV-1 infection into a chronic illness and has curtailed the morbidity of infected individuals. Unfortunately, HAART can have severe side effects. Additionally, new strains of HIV-1 are emerging that are resistant to suppressive treatments. In view of the increasing number of resistant HIV strains, new active agents are necessary and are currently under development.

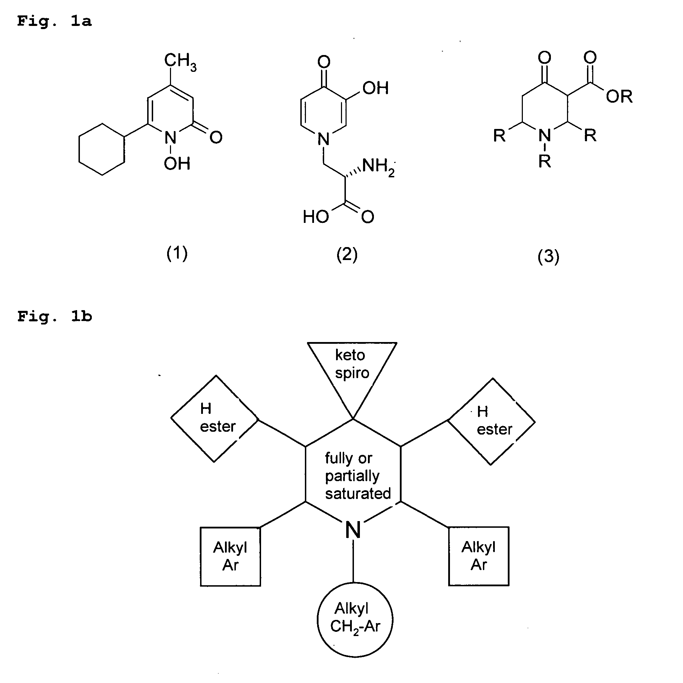

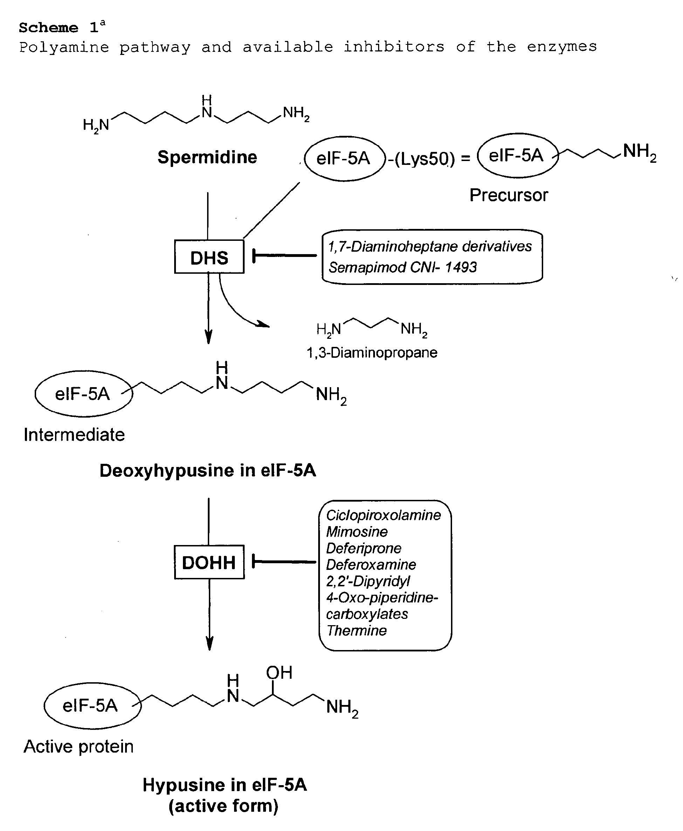

One potential further target for antiretroviral therapy is the biosynthesis of the eukaryotic initiation factor 5A (eIF-5A). eIF-5A is an essential cofactor of the HIV regulatory protein Rev and the HTLV regulatory protein Rex which are important for replication of the respective retroviruses. (¹ and Elfgang, C. et al. (1999). Evidence for specific nucleocytoplasmic transport pathways used by leucine-rich nuclear export signals. Proc. Natl. Acad. Sci. U. S. A 96, 6229-6234; Katahira,J. et al. (1995). Effects of translation initiation factor eIF-5A on the functioning of human T-cell leukemia virus type I Rex and human immunodeficiency virus Rev inhibited trans dominantly by a Rex mutant deficient in RNA binding. J. Virol. 69, 3125-3133.) The unusual amino acid hypusine (see Fig. 1) is a posttranslational modification of the eukaryotic initiation factor 5A (eIF-5A) and necessary for eIF-5A activity.² Hypusine is formed in two steps by deoxyhypusine synthase (DHS) [EC 1.1.1.249] and by deoxyhypusine hydroxylase (DOHH) [EC1.14.99.29].³ DHS transfers an aminobutyl moiety from the triamine spermidine to a specific lysine residue in the eIF-5A precursor protein to give deoxyhypusine⁴ and subsequently DOHH hydroxylates this molecule completing the hypusine biosynthesis.

The direct inhibition of enzymes in this pathway, adenosylmethionine decarboxylase (AdoMetDC), DHS and DOHH, with small molecules has already been shown to block the Rev activity and, thus, the virus replication^{5,6,7,8} indicating that inhibition of hypusine (especially DHS and DOHH) is a possible strategy for the development of anti-HIV drugs. Depletion of eIF-5A causes inhibition of cell growth, which also makes DOHH into an interesting target for drugs against proliferative diseases.

In fact, targeting of enzymes of this pathway like the ornithine decarboxylase (ODC) [EC 4.1.1.17], adenosylmethionine decarboxylase (AdoMetDC) [4.1.1.50], and the spermidine synthase (SPDS)[EC 2.5.1.16] turned out to be valuable for both antiparasitic chemotherapy and prevention.^{9,10,11}

Studies have shown that mature eIF-5A formation in plasmodia can be blocked by inhibition of DHS by means of 1,7-diaminoheptane *in vitro.*¹² Additionally, eIF-5A formation can be prevented by inhibition of DOHH. Findings in rat testis have demonstrated that the DOHH can be inhibited by the antifungal drug ciclopiroxolamine (Fig. 1). Ciclopiroxolamine showed antiangiogenetic effects in human vascular endothelial cells (HUVEC) and antiproliferative effects in the chick aortic arch sprouting assay.¹³ Moreover, ciclopiroxolamine inhibits the *in vitro* proliferation of the chloroquine sensitive (CQS) NF54 *P. falciparum* strain with an IC₅₀ value of 8.2 µM. However, ciclopiroxolamine was ineffective in vivo in a rodent malaria model. Previously, piperidone-type compounds have been shown to efficiently inhibit the DOHH, possibly by complexing the metal ion in the catalytic unit of the enzyme.

The plant amino acid L-mimosine (Fig. 1) can reversibly block mammalian cells at late G1-phase and leads to a notable reduction in the steady-state level of mature eIF-5A by means of DOHH inhibition.¹⁴ These findings prompted the determination of the antiplasmodial effect resulting in an IC₅₀ value of 32 µM for the chloroquine susceptible (CQS) strain and 39 µM for the chloroquine resistant (CQR) strain. However, mimosine was toxic in a rodent malaria model.

Since the 2- and 4-piperidone skeleton of ciclopiroxolamine and mimosine are putatively complexing the essential catalytic metal ion (iron),¹⁵ 4-oxo-piperidine-3-carboxylates (Fig. 1a), being perfect chelators of the metal either via the enolizable β-ketoester moiety (cf. acetylacetonate complexes of iron¹⁶) or via the three nitrogens, in position 1 and in the pyridines,^{16,17} were recently tested for the inhibitory properties of DOHH in *P. falciparum* and found to be more active than ciclopiroxolamine and mimosine.¹⁸ These findings proved the hypusine biosynthesis pathway to be a target for antimicrobial therapy¹⁹.

In light of this, there is a need in the art to develop novel agents capable of inhibiting DOOH. Such agents could be active against retroviruses, such as human immunodeficiency virus (HIV), e.g., HIV type 1 (HIV-1), or HTLV, by blocking the enzymes of the polyamine pathway (see Scheme 1). In order to find new lead compounds for the development of drugs against the aforementioned diseases, a library of piperidine derivatives was produced and tested.

The library of piperidines (see Fig. 1b) is composed of a variety of 4-oxo-piperidine-3,5-dicarboxylates and 3-monocarboxylates carrying different substituents in 2- and 4-position and on the nitrogen, of spiropiperidine compounds, and of piperidine oximes and related ethers.

The present invention provides a group of novel compounds active against pathogens. In particular, the present invention discloses a composition comprising a 4-Oxo-piperidine-carboxylate according to Formula I 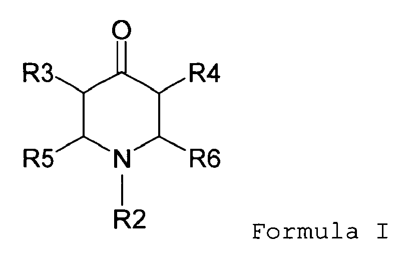 wherein R3 and R4 are selected from the group consisting of H, substituted or unsubstituted Alkyl and COOR1, wherein R1 is substituted or unsubstituted Alkyl, substituted or unsubstituted Alkylen or substituted or unsubstituted Aryl, with the proviso that at least one of R3 and R4 is COOR1,

R2 is selected from the group consisting of H, substituted or unsubstituted Alkyl, substituted or unsubstituted Alkylen, substituted or unsubstituted Alkin, or substituted or unsubstituted Aryl,

R5 and R6 are independently selected from the group consisting of substituted or unsubstituted Phenyl.

In the context of the present invention, Alkyl means any saturated hydrocarbon substituent, which can be linear or branched. Linear Alkyl has the general formula CₙH₂ₙ₊₁. An Alkyl may comprise 1-20, preferably 1 to 10 C atoms or 1 to 6 C-atoms. For example, Alkyl may be Methyl, Ethyl, Propyl, Isopropyl, Butyl, Isobutyl, Pentyl, Isopentyl or Hexyl.

In the context of the present invention, Alkylen means any unsaturated hydrocarbon substituent, which can be linear or branched. Linear Alkylen may have the general formula CₙH₂ₙ₋₁. An Alkylen may, e.g., comprise 2-20, preferably 2 to 10 C atoms or 2 to 6 C-atoms. Alkylen may comprise more than one double bond. For example, Alkylen can be Allyl (2-Propenyl, H₂C=CHCH₂-) or Vinyl (Ethenyl, H₂C=CH-).

In the context of the present invention, Alkin means any unsaturated hydrocarbon substituent, which can be linear or branched. Linear Alkin may have the general formula CₙHₙ₂ₙ₋₃. An Alkin may comprise 2-20, preferably 2 to 10 C atoms or 2 to 6 C-atoms.

In the context of the present invention, Aryl means any aromatic hydrocarbon substituent, for example, Phenyl, Benzyl, Tolyl, Xylyl or Naphtyl. Aryl (Ar) comprises heteroaryl, wherein the aromatic ring system may comprise one or more identical or different heteroatoms, e.g., Pyridyl, Pyrazinyl, Pyrimidinyl, Pyradazinyl, Indolyl, Purinyl or Chinolyl. Pyridiyl may be, e.g., 2-Pyridyl.

In the context of the present invention, substituted means that one or more hydrogen(s) of the group is(are) substituted by Alkyl, Alkylen, Alkin, Aryl, Alkanol, Hydroxy, Carboxy, Nitro or Halogen (Fluor, Chlor, Brom or Iod) or Ether(-O-Alkyl, O-Alkylen, O-Alkin or O-Aryl). Preferably, substituents comprise less than 50, less than 20, less than 10 or less than 5 atoms. For example, substituted Phenyl may be Benzyl, Nitrophenyl.

It has been shown that compositions comprising the compounds of the invention, wherein R5 and/or R6 are independently selected from the group consisting of substituted or unsubstituted Phenyl, are especially advantageous for the preparation of medicaments for treatment of retroviral diseases, proliferative diseases and trypanosomiasis.

In one embodiment of the invention, R5 and/or R6 is/are substituted or unsubstituted or substituted or unsubstituted Nitrophenyl. Nitrophenyl may be selected from the group comprising 4-NO₂-phenyl, 3-NO₂-phenyl and 2-NO₂-phenyl. R5 and R6 may be identical or different. Nitrophenyl may be further substituted, e.g., with Alkyl, in particular, Methyl, Ethyl or Propyl.

The compound of the present invention is a 4-oxo-piperidine-carboxylate, i.e., either R3 or R4 or both are an ester (COOR1). It may thus be a monocarboxylate or a dicarboxylate, wherein R3 and R4 are COOR1. R1 may be Alkyl, Alkylen, Alkin or Aryl, e.g., Methyl, Ethyl, Propyl, Butyl, Isobutyl, Pentyl, Hexyl, Allyl, Vinyl or Phenyl. This group may again be substituted. COOR1 may also represent an ester of a fatty acid.

Preferably, R2 is selected from the group consisting of Allyl and substituted or unsubstituted Phenyl. Substituted Phenyl, in particular substituted Phenyl as R2, may be selected from the group comprising Benzyl, 4-Chlorbenzyl, 4-Methoxybenzyl and 4-Methylbenzyl. Interestingly, it has been shown that introduction of an alkyl substituted with a Hydroxy or Carboxy group as R2 tends to lead to a higher toxicity against macrophages. Compounds with higher toxicity are more suitable for in vitro than for in vivo uses, e.g. for experiments regarding the relevance of the enzyme DOOH for different cells and pathogens.

In one embodiment of the invention, R3 and R4 are COOR1 or H, R1 is Ethyl, R2 is Allyl and R5 and R6 are Nitrophenyl. In particular, R5 and R6 are selected from the group comprising 4-NO₂-phenyl and 3-NO₂-phenyl.

The library of compounds that was synthesized was tested for *in vitro* and *in vivo* activity against bacteria, viruses and parasites, e.g., *Trypanosoma brucei brucei* and bacteria, such as *Staphylococcus* aureus and *Staphylococcus epidermidis, Escherichia coli* and *Pseudomonas aeruginosa* and assays for antiviral activity such as an inhibition assay of HIV-1 replication. Finally, active compounds were tested for their capacity to inhibit the growth of the macrophage cell line J774.1. Applying this strategy resulted in the finding of lead structures for the development of small compounds of selective activity against one of the aforementioned diseases having low cytotoxicity.

It was found that the compounds of the invention were effective both against retroviruses and trypanosomatoids. Trypanosomatids such as *Trypanosoma brucei gambiense* and *T. b. rhodesiense* are the causative agents of African sleeping sickness, *T. cruzi* of South American Chagas' disease. Both diseases are referred to as trypanosomiasis in the context of the invention. Worldwide, millions of people are infected by these parasites and tens of thousands of patients die every year.²⁰ The chemotherapy against all these diseases is very limited and unsatisfactory due to the emerging levels of resistance against plasmodicidal drugs and the lack of safe drugs against all forms of trypanosomiasis.²¹ Even faced by an increasing number of infections with resistant and multiresistant pathogens, the major pharmaceutical companies are mostly neglecting this urgent medical problem and are frequently leaving the field of antiinfective drug discovery, especially in the case of tropical diseases.

The present invention thus also provides a pharmaceutical composition comprising the composition of the invention and a pharmaceutically suitable excipient. In the context of the invention, a pharmaceutical composition (medicament) is useful for treatment or alleviation of a disease or disorder in humans and/or animals. The excipient may be a carrier. The pharmaceutical composition may be formulated, e.g., for oral or intravenous administration. The pharmaceutical composition may be liquid or solid or in gel form. In particular, water, ethanol and/or DMSO may be used as an excipient. In the context of the present invention, "a" is not to be construed as limited to "one", i.e., for example, more than one excipient may be used, or more than one compound of the invention may be used. Further active ingredients may be incorporated in the pharmaceutical composition or administrated in combination with it. For example, other antiviral agents, such as those mentioned above, may be combined with the pharmaceutical composition of the invention for an antiretroviral medicament, or a tyrosine kinase inhibitor may be combined with the pharmaceutical composition of the invention for medicament for treatment of a proliferative disease.

The present invention provides the use of a composition comprising a 4-oxo-piperidine-carboxylate according to Formula I 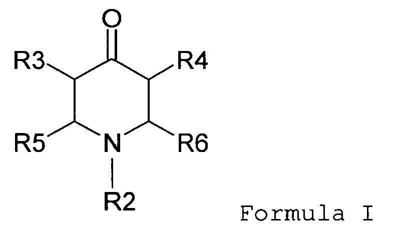 wherein R3 and R4 are selected from the group consisting of H, substituted or unsubstituted Alkyl and COOR1, wherein R1 is substituted or unsubstituted Alkyl, substituted or unsubstituted Alkylen or substituted or unsubstituted Aryl, with the proviso that at least one of R3 and R4 is COOR1,

R2 is selected from the group consisting of H, substituted or unsubstituted Alkyl, substituted or unsubstituted Alkylen or substituted or unsubstituted Aryl,

R5 and R6 are independently selected from the group consisting of Aryl, in particular substituted or unsubstituted Phenyl or Pyridine,

for the preparation of a pharmaceutical composition for treatment of a retroviral disease, in particular HTLV or HIV, e.g., HIV-1, a proliferative disease or trypanosomiasis. A proliferative disease is a disease associated with abnormal proliferation of cells, in particular a hematologic neoplasia, especially leukemia, e.g., chronic myeloid leukemia (CML). Trypanosomiasis may be Chagas' disease or sleeping sickness.

In one embodiment, the composition thus used is the composition wherein R5 and/or R6 are substituted or unsubstituted Phenyl, as described above. Alternatively, R5 and/or R6 may be Pyridine, in particular 2-Pyridine.

The invention also provides a method for the treatment of a retroviral disease, in particular HTLV or HIV, a proliferative disease, or trypanosomiasis, wherein a composition comprising an effective amount of 4-oxo-piperidine-carboxylate according to Formula I 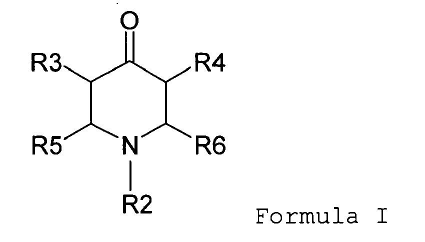 wherein R3 and R4 are selected from the group consisting of H, substituted or unsubstituted Alkyl and COOR1, wherein R1 is substituted or unsubstituted Alkyl, substituted or unsubstituted Alkylen or substituted or unsubstituted Aryl, with the proviso that at least one of R3 and R4 is COOR1,

R2 is selected from the group consisting of H, substituted or unsubstituted Alkyl, substituted or unsubstituted Alkylen or substituted or unsubstituted Aryl,

R5 and R6 are independently selected from the group consisting of Aryl, in particular substituted or unsubstituted Phenyl or Pyridine,
is administered to a patient. A patient can be a human or animal subject in which the presence of retroviruses, trypanosomes or a proliferative disease has been diagnosed.

It has been found to be especially advantageous to use the composition for treatment of trypanosomiasis, wherein R3 or R4 is H, R5 and R6 are 2-Pyridine and R2 is selected from the group comprising Benzyl, 4-Methylbenzyl, 4-Chlorbenzyl, 4-Methoxybenzyl and Allyl. In one embodiment, R3 and R4 are COOR1, R1 is Ethyl or Methyl, R2 is selected from the group consisting of Allyl, Benzyl or 4-Methyoxybenzyl, R5 and R6 are selected from the group consisting of 4-NO₂-phenyl and 3-NO₂-phenyl and the composition is used for treatment of trypanosomiasis.

In another preferred embodiment, R1 is Ethyl or Methyl, R2 is selected from the group consisting of Allyl and Benzyl, R5 and R6 are 4-NO₂-phenyl, and the composition is used for treatment of HIV, HTLV or proliferative diseases. Preferably, R3 and R4 are COOR1 and R1 is Ethyl, R2 is Allyl, and the composition is used for treatment of HIV.

The present invention provides compounds that may be used for treatment of the mentioned diseases and for experimental purposes. These may be purely scientific in purpose, like elucidation of biochemical pathways and the mode of interaction with DOHH or directed towards in vitro inhibition of the growth of pathogens or hyperproliferative cells, but they may also be directed towards development of medicaments with even higher activity against the pathogens or against proliferative diseases and/or with less toxicity and better tolerability. Thus, the highly active compounds provided herein may be used as lead compounds for development of even better medicaments.

For example, the 3,5-diethyl piperidone 3,5-dicarboxylates **11** and **13** (cf. Table 1 below) being substituted with 4-nitrophenyl rings in 2- and 6-position were found to be highly active against *Trypanosoma brucei brucei* and to have a low cytotoxicity against macrophages. The corresponding monocarboxylates are also very active against *T. brucei brucei.* The dichlorobenzyl ether of the piperidine oxime **53** and compound **11** were best able to inhibit replication of HIV-1. Thus, these compounds are promising leads for future drug development to combat parasites and retroviral infections.

In one embodiment, the present invention provides a method for identifying a composition having activity against retroviruses, e.g., HIV, HTLV, or trypanosomes or antiproliferative activity, comprising synthesizing the composition of the invention and testing said composition for its activity, optionally comprising repeating synthesis with substituted or modified reactants and repeating testing. Preferably, the compounds thus identified have a high activity, i.e., an activity in a submicromolar or lower micromolar range (up to 10 µM, preferably up to 5 µM) in the assays described below. Preferably, the compounds also have a low toxicity. This may, for example, be tested in the macrophage cytotoxicity assay disclosed below (ED₅₀ higher than 100 µM). Preferably, the therapeutic index is high, i.e., the ED₅₀ in the toxicity assay is more than 10 times higher, 50 times higher or 100 times higher or 200 times higher than the dose effective against the pathogen or disease. Of course, activity and toxicity may also be tested in animal models or clinical studies according to methods known in the art.

Substituted or modified reactants may lead to compounds wherein, in comparison to the compounds explicitely described herein, e.g., small substituents are added or substituents are exchanged with chemically similar groups. For example, two polar groups or non-polar groups, respectively, may be exchanged. Protonable or deprotonable as well as methyl groups may be introduced instead of hydrogen or ethyl, Vinyl may be introduced instead of Allyl, Benzyl may be introduced instead of Phenyl, or vice versa, or the position of substituents, e.g., in a ring system, may be rearranged.

The invention teaches a method for the preparation of a pharmaceutical composition for treatment of a retroviral disease, e.g., HTLV or HIV, a proliferative disease or trypanosomiasis, comprising carrying out said method for identifying a composition having activity against retroviruses, e.g., HIV, HTLV, or trypanosomiasis or antiproliferative activity, selecting a composition with high activity and low toxicity and formulating said composition with a pharmaceutically suitable excipient.

The invention is further described with regard to specific examples for the purpose of illustration. The examples should however not to be construed as limiting. All references cited are herewith explicitly incorporated herein.

### EXAMPLES

### Chemistry

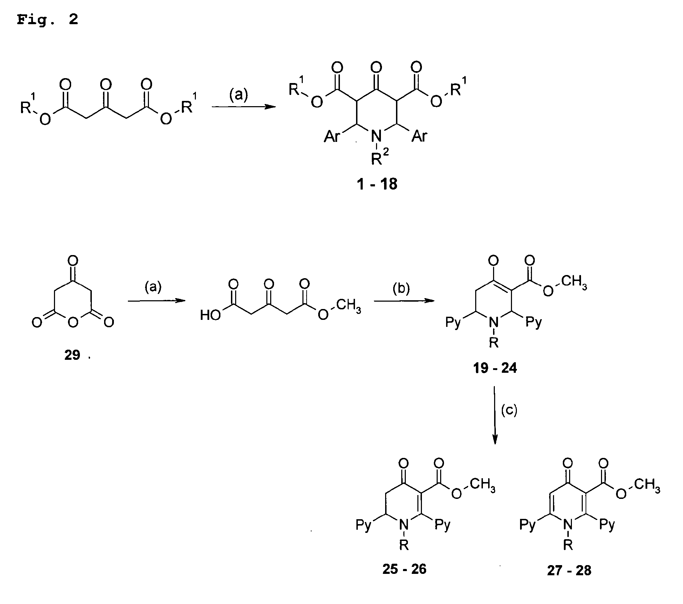

The 4-oxo-piperidine 3,5-dicarboxylates **1 - 18** (see Fig. 2) were achieved via a double Mannich condensation of one mole of a corresponding primary amine, two moles of pyridine-2-carbaldehyde and 3- or 4-nitrophenylcarbaldehyde, respectively, and dimethyl oxoglutarate in analogy to Merz and Haller,²² Ashauer-Holzgrabe and Haller,²³ and Kuhl et al.²⁴ The present invention provides a method for the preparation of the composition of the invention comprising a novel 4-Oxo-piperidine-carboxylate, wherein an amine, pyridine-2-carboxaldehyde or a nitrobenzaldehyde are reacted with an oxoglutarate.

Different substituents were introduced in the piperidine rings of the dicarboxylates, e.g. alkylcarboxylate and alkanol residues attached to the nitrogens, and, e.g., nitro groups were attached to the aromatic ring in 2- and 6-positions (Tab. 1a). Especially the latter variation led to compounds of higher activity.

The analogous monocarboxylates **19 - 24** (Tab. 1b) can be obtained by conversion of 2,4,6-trioxotetrahydropyrane, formed by solvolytic cleavage with methanol, with freshly destilled pyridine-2-carbaldehyde and the correspondingly substituted primary amines (cf. ref.²⁵ ). The monocarboxylates were always isolated in the enol form. The corresponding dihydropyridine and tetrahydropyridine compounds **25 - 28** (see Fig. 2) can be achieved by means of an oxidation using cerium (IV) sulfate.^{22,23,25}

In order to obtain the spiropiperidine **33 - 41**, and **44, 45** and **47** the corresponding 4-oxo-piperidine-3,5-dicarboxylates 30a-d have to be synthesized via a double Mannich condensation of ammonium bromide, two moles of the corresponding freshly destilled aliphatic aldehyde and dimethyl oxoglutarate. The dicarboxylates were isolated as a mixture of keto-enol tautomers mostly occurring as trans-isomers with regard to the alkyl groups. Ester hydrolysis and decarboxylation was performed with concentrated hydrochloric acid to give the 2,6-alkyl substituted and non-substituted 4-piperidones **31** which are mainly isolated as cis-isomers indicating a configurational change of the alkyl groups during the course of the reaction which is in accordance with finding of Siener et al.²⁶ N-Benzylation can be achieved by means of benzylbromide in acetonitrile in presence of an excess of potassium carbonate resulting in high yields of the piperidone **42**. Via a Strecker synthesis²⁷ the nitrile compounds **32a-f** and **43a,b** were synthesized in glacial acid using trimethylsilylcyanide, and aniline and benzylamine, respectively, as amine components. In order to avoid the hydrolysis of the amine, the reaction was stopped by addition of a concentrated ammonia solution. Only the cis isomer of all compounds was isolated, because in the case of the trans-isomer the carbonyl group is difficult to attack due to one axial alkyl group. Conversion of the compounds **32a-f** and **43a,b** with chlorosulfonylisocyanates and subsequent refluxing in 1 M hydrochloric acid leads directly to the spiro derivatives **33-36** and **44**. The nitrogen N3 in the imidazolidione skeleton can be selectively alkylated to give **37-41** and **45**. The spiro compound **47** was obtained via the amide **46** being formed by conversion with concentrated sulfuric acid and working-up with concentrated ammonia solution. Ring closure was achieved with formamide at 200 °C in analogy to Röver et al.²⁸ In contrast to Röver et al. the reduced compound **47** was forthwith isolated. Thus, the reduction step with NaBH₄ was dispensable.

The N-alkylated oxime of the 4-piperidone **48** was synthesized starting from the piperidone which can be alkylated with phenylpropylbromide. Subsequently the ketone can be converted to the oxime by using hydroxylamine hydrochloride. **48** was alkylated with dichlorobenzylbromide to give the oxime ether **49.**

The synthesis of the corresponding 4-piperidinecarbaldehyde oximes started off from pyridine-4-carbaldehyde whose aldehyde function was firstly protected by means of ethylenglycol. The resulting acetal was N-alkylated with phenylpropylbromide in the microwave within 2 h to give compound **50** which can be hydrogenated with PtO₂/H₂ to **51**. Acetal cleavage and subsequent oxime formation with hydroxylamine hydrochloride gave the piperidone oxime **52** which was alkylated again with dichlorobenzylchloride to yield the oxime ether **53**.

### Results and Discussion

All piperidine compounds were subjected to the mentioned microbiological assays. None of the compounds showed considerable activity against the bacteria S. *aureus, S. epidermidis, P. aeruginosa* and the fungi C. *albicans* (data not shown). Additionally, none of the compounds inhibited the formation of biofilm produced by S. *aureus* (data not shown).

The inhibition activity against *T. brucei brucei* is summarized in Table 1 as well as the toxicity against macrophages after 48 h. Structure-activity relationships (SAR) will be qualitatively discussed chemical group by group.

Many of the piperidone dicarboxylates show activity against *T. brucei brucei .* The activity (IC₅₀ and ED₅₀ values) fell in the higher micromolar range of concentration if the piperidones are armed with pyridines rings in 2- and 6-position **(1-7)** and regardless of the substitution on the nitrogen. The compounds having a benzylic moiety (i.e. benzyl for **9** and pyridinylmethyl for **8**) show a higher activity against *T. brucei brucei.* However, the effective dosis for parasites and for the toxicity against macrophages are close together.

The replacement of the pyridine rings with 4-nitrophenyl and with 3-nitrophenyl rings enhances the activity against *T. brucei brucei* considerably in connection with low cytotoxicity against macrophages. In addition, these compounds are much better soluble in water and buffer than the pyridine substituted analogues. Especially the diethyl dicarboxylates **11** and **13**, still better than the corresponding dimethyl dicarboxylates **10** and **12**, show activity against parasites in the lower micromolar concentration range with concomitant very low cytotoxicity and can thus be regarded as lead compounds for further improvement. The 3-nitrophenyl substituted compounds are also active against *T. brucei brucei.*

In a previous study the corresponding 3-monocarboxylate 4-piperidones **21** and **24** have shown high activity against the plasmodia after 48 h. A series of compounds was synthesized having a varying substitution attached to the nitrogen. The monocarboxylates are amongst the compounds with the highest activity against *T. brucei brucei.* With exception of the N-3-methoxybenzyl substituted compound **23**, the trypanocidal activity was in the lower and submicromolar range of concentration, more or less independent from substitution of the benzyl substituent. Since the cytotoxicity in macrophages was found to be hundred times higher than the trypanocidal activity, these compounds can be regarded as leads for the development of trypanocidal drugs.

Since mimosine and ciclopiroxolamine can be regarded as dihydropyridine derivatives, structurally corresponding compounds were considered in our library. The trypanocidal activity of all compounds **25-28** is rather low. Since these compounds are difficult to isolate from oxidation of the corresponding piperidone, we did not embark on this strategy. Without being bound by the theory, the lack of activity may be due to the fact that the β-ketocarboxylate is not enolizable because of double bonds next to this moiety. Thus, a complexation of the DOHH via the metal ion might be difficult.

Some of the spiro compounds that were synthesized show an interestingly high trypanocidal activity being in the same concentration range as eflornitine. Especially a phenylalkyl substitution in position N3 of the imidazolidione ring seems to be advantageous. With increasing length of the alkyl spacer, the inhibitory activity increases (cf. **38, 40** and **41**). The activity can be enhanced by a nitrobenzyl substitution resulting in **39**, a compound with the highest activity within this series and a therapeutic index of 10.

Due to the hypothesis that compounds complexing metal ions are able to inhibit the DOHH and to show antiplasmodial activity, oximes with piperidone moiety (**48** and **52**) were synthesized in addition to the corresponding dichlorobenzyl ethers (**49** and **53**). Interestingly, neither the oximes **48** and **52** nor the oxime ether **49** are active against trypanosomes. In contrast, the oxime ether of the piperidine aldehyde **53** is active against trypanosomes. **53** will be a new lead compound for further development. Since **53** is not able to form a complex with metal ions, its effect is surprising, and the mode of action has to be elucidated in the future.

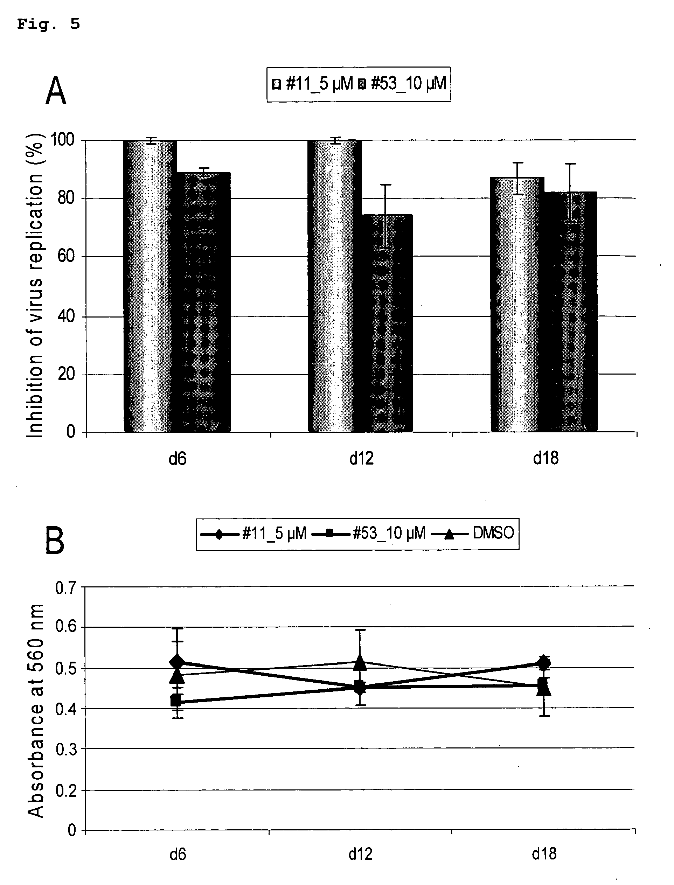

Since the host cell eIF-5A, a co-factor of the HIV protein Rev and of the HTLV protein Rex, is involved in virus replication, the inhibition of DOHH and DHS will reduce the multiplication rate of the virus. Consequently, active compounds, e.g., compounds **11 - 13** and **53** were tested against the R5-tropic HIV-1 strain *BaL* and/or against the X4-tropic HIV-1 strain *NL4*/*3* in the human T cell line PM1. With compounds **12** and **13,** 20% inhibition of virus replication was observed. Surprisingly, compound **11** was able to completely inhibit replication of HIV-1 *BaL* at a concentration of 5 µM and HIV-1 *NL4*/*3* at a concentration of 6 µM as compared to the respective control cultures (which were treated with the drug-solvent DMSO). When HIV-1 *BaL* infected PM1 cells were exposed to a concentration of 10 µM of compound **53,** virus replication was inhibited by 85% (Fig. 5). Finally, the parallel analysis of cellular metabolic activities revealed that these antiviral effects were not caused by deleterious effects of the respective drugs on the host cell.

### Conclusion '

The evaluation of the library revealed several promising effective compounds, which may also be used as lead compounds for further drug development, e.g. the 3,5-diethyl piperidone 3,5-dicarboxylates **11** and **13** and the corresponding monocarboxylates. In particular, the compounds **11** and **53** appear to be promising leads for anti-HIV-1 drug development. In addition to the good anti-infective activity, the main advantage of these compounds is the simple synthesis route. Also, the compounds were satisfyingly water soluble for biological evaluation.

The antimicrobial activity can be further increased by modifications of the disclosed compounds, but also the cytotoxicity, especially of compound 53, may be reduced by variation of the substitution pattern.

### Experimental

### Material and Methods

Melting points were determined on a model B 540 Büchi or a Sanyo Gallenkamp melting point apparatus (Sanyo Gallenkamp, UK) and are uncorrected. ¹H NMR and ¹³C NMR spectra were recorded with a Bruker AV 400 spectrometer (¹H, 400.132 MHz; ¹³C, 100.613 MHz). Chemical shifts (δ) are expressed in ppm and coupling constants (J) in Hertz. Abbreviations for data quoted are: s, singlet; d, doublet; t, triplet; m, multiplet; br, broad. The center of the peaks of CDCl₃ and DMSO-*d*₆ was used as internal reference. FT-IR spectra were recorded on a Bio-Rad PharmalyzIR equipped with an ATR unit. TLC analyses were performed on commercial silica gel 60 F₂₅₄ aluminum sheets; spots were further evidenced by spraying with a dilute alkaline potassium permanganate solution. Microanalyses (C, H, N) of the new compounds agreed with the theoretical value within ± 0.4%.

The dimethyl 4-oxo-piperidine-3,5-dicarboxylates **1** and **2** were prepared according to ref. 24, the methyl 4-oxo-piperidine-3-monocarboxylates **20, 21** and **24**, and the dihydro- and tetrahydropyridine compounds **25 - 28** were synthesized according to ref. **18.**

### Synthesis

### General procedure for the synthesis of the 3,5-dialkyl 2,6-di-2-aryl-4-oxo-piperidine 3,5-dicarboxylates 1-18 and 30 a-d, modified after²⁹

0.02 mol of the corresponding amine, 0.04 mol of pyridine-2-carboxaldehyde, and m- and p-nitrobenzaldehyde, respectively, were dissolved in 20 mL methanol and cooled to 0°C. Over a course of about 1 h 0.02 mol of dimethyl oxoglutarate and diethyl oxoglutarate, respectively, were dropwise added. The solution was allowed to stand overnight at 5°C. The product was obtained by filtration of the precipitate formed. In the case no precipitate appeared, the solvent was removed *in vacuo* at 40 to 50°C and the remaining oil dissolved in methanol/diethyl ether or treated with diethyl ether. The obtained crystals could be washed with a mixture of methanol/diethyl ether and recrystallized from methanol. The piperidones could be isolated in yields ranging from 28 to 81 %.

**Dimethyl (2*R*,6*S*)-4-oxo-2,6-di(pyridin-2-yl)piperidine-3,5-dicarboxylate (1)** C₁₉H₁₉N₃O₅ (369.4 g/mol), yield: 33 % (67 %) ; **¹H NMR** (DMSO-d₆, δ = ppm, *J* = Hz) 2.94 (t, 1H, *³J = 12.3,* N*H) ;* 3.53 (s, 6H, COOC***H₃***); 4.25 (d, 2H, *³Jₐₐ = 10.5,* C***H***C=O); 4.62 (dd, 2H, ³*J* = *12.3,* ³*Jₐₐ =10.5,* C***H***NH); 7.32 (ddd, 2H, *J = 1.0, J* = *4.8, J = 7.6, **H6'*** or ***H4**'*); 7.45 (d, 2H, *J = 7.8, **H6'*** or ***H4'***) ; 7.78 (dt, 2H, *J* = 7. 6, *J* = *1.8, **H5'***)**;** 8.50-8.56 (m, 2H, ***H3'***)*.* **¹³C NMR** (DMSO-d₆, δ = ppm) 51.6 (COO***C***H₃); 61. 6 (***C***HC=O); 63.0 (CHNH); 123.2 (***C4'*/*C6'***); 137.0 *(**C5**');* 148.9 *(**C3**');* 158.0 (***C1'***); 168.6 (***C***OOCH₃); 202.1 (***C***=O). **IR** (cm⁻¹) 3296; 3021; 2954; 1739; 1701; 1432; 1335; 1213; 1110; 816; 776. mp. 175 °C (170-171 °C).²³

**Dimethyl (2*R*,6*S*)-1-allyl-4-oxo-2,6-di(pyridin-2-yl)piperidine-3,5-dicarboxylate (2)** C₂₂H₂₃N₃O₅ (409.5 g/mol), yield: 87 % ; **¹H NMR** (CDCl₃, δ = ppm, J = Hz) 3.00 (m, 2H, NC***H***₂); 3.59 (s, 3H, COOC***H***₃); 3.74 (s, 3H, COOC***H***₃); 4.18 (d, 1H, J = 10.3, C***H***C=O); 4.68 (d, 1H, *J* = 10.3, C***H***NH); 4.97 (s, 1H, NC***H***Cq); 5.19 (ddd, 2H, J = 17.3, J = 10.1, J = 1.2, =C***H***₂), 5.79 (m, 1H, J = 17.3, J = 10.1, NCH₂C***H***=); 7.07- 7.19 (m, 2H, ***H_{aromat.}***); 7.29 (m, 1H, ***H_{aromat.}***)**;** 7.52- 7.71 (m, 3H, ***H_{aromat.}***); 8.43 (m, 1H, ***Hₐᵣₒₘₐₜ.***); 8.60 (m, 1H, ***H_{aromat.}***); 12.52 (s, O***H***). **¹³C NMR** (CDCl₃, δ = ppm) 44.32 (***C***HC=O); 50.53 (N***C***H₂); 51.76, 52.5 (COO***C***H₃); 59.43 (***C***HNCH₂); 60.38 (H₂CN***C***H); 97.78; 117.68 (=***C***H₂); 121.84; 122.20; 122.76; 123.06 (***C4'*/*C6'***); 135.86 (NCH₂***C***H=); 136.02; 136.12 *(C5');* 148.13; 148.83 *(C3');* 158.24 (***C1'***); 161.32 (***C1**'*); 167.33 (***C***OOCH₃); 171.12, 172.05 (***C***OOCH₃). **IR** (cm⁻¹) 3040; 3000; 1720; 1650; 1430; 1360; 1250; 1005; 980; 825; 770. mp. 133 °C (134 °C).²³

**Dimethyl (2R,6S)-1-(2-hydroxyethyl)-4-oxo-2,6-di(pyridin-2-yl)piperidine-3,5-dicarboxylate (3)** C₂₁H₂₃N₃O₆ (413.4 g/mol), yield: 55 % ; **¹H NMR** (CDCl₃, δ = ppm, J = Hz) 2.58-2.68 (m, 2H, CH₂C***H***₂OH); 3.35-3.43 (m, 1H, C***H***₂CH₂OH); 3.63 (s, 3H, CqCOOC***H***₃); 3.67-3.76 (m, 1H, C***H***₂CH₂OH); 3.74 (s, 3H, CHCOOC***H***₃); 4.26 (d, 1H, *J =* 10.6, C**H**C***H***COOCH₃); 4.46 (s, 1H, CH₂CH₂O***H***); 4.55 (d, 1H, J = 10.6, C***H***CHCOOCH₃) 5.03 (s, 1H, CqC***H***); 7.08-7.14 (m, 2H, ***H3**"* and H5"); 7.19-7.25 (m, 1H, ***H5*'**); 7.33 (d, 1H, J = 7.8, ***H3'***); 7.55 (td, 1H, J = 7.7, J = 1.8, ***H4"***)**;** 7.69 (td, 1H, J = 7.7, J = 1.8, ***H4'***); 8.40-8.47 (m, 1H, ***H6"***); 8.65-8.72 (m, 1H, ***H6'***)*.* **¹³C NMR** (CDCl₃, δ = ppm) 44.4 (CH***C***HCOOCH₃); 48.6 (CH₂***C***H₂OH); 52.0 (CqCOO***C***H₃); 52.7 (CHCOO***C***H₃); 59.3 (***C***HCHCOOCH₃); 60.2 (***C***H₂CH₂OH); 61.2 (Cq***C***H); 97.1 (***Cq***=C-OH); 121.8 (***C3'***); 122.4 (***C5**'*); 1227, 124.1 (***C3"*/** ***C5"**);* 136.6 (***C4'*/ *C4"***); 148.4 (***C6"***); 149.8 (***C6'***); 157.5, 161. 6 (***C2'*/** ***C2"**)*; 169.6 (Cq=***C***OH); 171.3, 172.1 (***C***=O). IR (cm⁻¹) 3333; 2930; 1726; 1437. mp. 135 °C.

**Dimethyl (2R,6S)-1-(2-hydroxypropyl)-4-oxo-2,6-di(pyridin-2-yl)piperidine-3,5-dicarboxylate (4)** C₂₂H₂₅N₃O₆ (427.4 g/mol), yield: 39 % ; keto-enol ratio: 1:3. **¹H NMR** (CDCl₃, δ = ppm, *J* = Hz) 1.49 (qu, 2H, J = 6.8, CH₂C***H***₂CH₂OH, ketone); 1.59 (qu, 2H, *J* = 6. 7, CH₂C***H***₂CH₂OH, enol); 2.23-2.35 (m, 2H, C***H***₂CH₂CH₂OH, ketone); 2.23-2.35, 2.42-2.49 (2m, each 1H, C***H***₂CH₂CH₂OH, enol); 3.15-3.30 (m 2H, CH₂CH₂C***H***₂OH, ketone); 3.15-3.30, 3.36-3.42 (2m, each 1H, CH₂CH₂C***H***₂OH, enol); 3.59 (2s, each 3H, COOC***H***₃, enol); 3.64 (s, 6H, COOC**H**₃, ketone); 4.10 (dd, 1H, *J = 0. 8, J =* 10.6, C***H***COOCH₃, enol); 4.25 (d, J = 6.6, C***H***COOCH₃, ketone); 4.27-4.32 (m, 1H, CH₂O***H***, enol); 4.37 (d, 1H, J = 10.6, NCHCH, enol); 4.73 (d, 2H, J = 6.6, NCHCH, ketone); 4.93 (s, 1H, NC***H***Cq, enol); 7.22-7.36 (m, 2H, ***H3"*** and ***H5"***, enol, 1H, ***H5'**,* enol, 2H, H5, ketone); 7.49 (d, 2H, J = 7.8, ***H3***, ketone); 7.67 (d, 2H, J = 8.1, ***H3',*** enol); 7.73 (td, 1H, J = 7.7, J = 1.9, ***H4"***, enol); 7.82-7.89 (m, 2H, H4, ketone, 1H, ***H4'*,** enol); 8.39-8.44 (m, 1H, ***H6*"**, enol); 8.49-8.54 (m, 2H, H6, ketone, 1H, *H6',* enol); 12.21 (s, 1H, C=C-OH). **¹³C NMR** (CDCl₃, δ = ppm) 30.6 (CH₂***C***H₂CH₂OH, ketone); 31.4 (CH₂***C***H₂CH₂OH, enol); 42.7 (***C***HC-OH, enol); 43.7 (N***C***H₂CH₂CH₂OH, enol); 44.6 (N***C***H₂CH₂CH₂OH, ketone); 51.6 51.7 (O***C***H₃, enol); 52.0 (O***C***H₃, ketone); 56.5 (***C***HC=O, ketone); 58.3 (CH₂CH₂***C***H₂OH, ketone); 58.7 (CH₂CH₂***C***H₂OH, enol); 59.3 (NCHCH, enol); 60.0 (N***C***HqC, enol); 63.2 (NCHCH, ketone); 98.0 (***Cq***=COH, enol); 122.2, 122.5, 122.7, 123.0 (***C3'*/ *C3", C5'*/** *C5",* enol); 123.0, 123.4 (***C3, C5,*** ketone); 136.6, 136.7 (***C4'***/ ***C4",*** enol); 137.1 *(C4,* ketone); 148.0; 148.3 (***C6'*/ *C6 ",*** enol); 148.5 *(C6,* ketone); 157.4 (***C2*,** ketone); 161.4, 157.9 (***C2'*/** ***C2"**,* enol); 166.2 (**C**OOCH₃, ketone); 169.0 (Cq=C-OH, enol); 170.6, 171.8 (**C**OOCH₃, enol); 198.6 (**C**=O, ketone); IR (cm⁻¹) 3524; 2957; 1736; 1438. mp. 136 °C.

**Dimethyl (2*R*,6*S*)-1-[2-(2-hydroxyethoxy)ethyl]-4-oxo-2,6-di(pyridin-2-yl)piperidine-3,5-dicarboxylate (5) :** C₂₃H₂₇N₃O₇ (457.5 g/mol), yield: 63 %; **¹H NMR** (CDCl₃, δ = ppm, J = Hz) 2.44- 2.51 (m, 1H, ); 2.67- 2.76 (m, 1H, NCH₂C***H***₂); 3.44- 3.58 (m, 3H, NC***H***₂CH₂OC***H***₂); 3.60 (s, 3H, COOC***H***₃); 3.75 (s, 3H, COOC***H***₃); 3.77- 3.80 (m, 2H, ); 3.62- 3.69 (m, 1H, ); 4.20 (d, 1H, J = 10.6, O=CC***H***COH); 4.59 (d, 1H, J = 10.6, CHNCH); 5.41 (s, 1H, NC***H***Cq); 7.08 (t, 1H, ***H4*"**); 7.22- 7.29 (m, 2H, ***H6"*/ *H4'***); 7.45 (d, 1H, ***H6'***) ; 7.53 (dt, 1H, ***H5*"**); 7.72 (t, 1H, ***H5'***); 8.42 (d, 1H, ***H3"***) ; 8.68 (d, 1H, **H3**"); 12.57 (s, O***H***). **¹³C NMR** (CDCl₃, δ = ppm) 45.29 (O=C***C***HCOH); 48.63 (N***C***H₂CH₂); 53.37, 53.96 (COO***C***H₃); 60.97 (CHNCH) ; 62.74 (NCH₂CH₂OCH₂***C***H₂OH); 64.36 (N***C***HCq); 73.18 (NCH₂***C***H₂); 73.98 (NCH₂CH₂O***C***H₂CH₂OH); 98.83 (***C***qCOH); 123.82 (***C6**'*); 123.87 (***C4*'**); 123.94 *(**C6**");* 125.20 (***C4"***); 137.67 (***C5'***); 138.38 (***C5"***); 149.57 (***C3*"**); 150.61 *(C3');* 159.61 (***C1'***); 162.15 (***C1"***); 170.17 (***Cq***OH); 173.02, 173.58 (***C***OOCH***₃***). **IR** (cm⁻¹) 3020; 3000; 2830; 1610; 1520; 1370; 1117; 821; 740. mp. 175°C.

**4-[(2R,6S)-3,5-bis(methoxycarbonyl)-4-oxo-2,6-di(pyridin-2-yl)piperldin-1-yl]butanoic acid (6)** : C₂₃H₂₅N₃O₇ (455.5 g/mol), yield: 70%; **¹H NMR** (CDCl₃, δ = ppm, J = Hz) 1.71- 1.83 (m, 1H, NCH₂C***H***₂), 1.89- 2.40 (m, 2H, NCH₂C***H***₂C***H***₂), 2.28- 2.47 (m, 2H, NC***H***₂CH₂C***H***₂), 2.51- 2.61 (m, 1H, NC***H***₂), 3.61 (s, 3H, COOC***H***₃); 3.74 (s, 3H, COOC***H***₃); 4.23 (d, 1H, J = 10.6, C***H***C=O); 4.52 (d, 1H, J = 10.6, CHNH); 5.06 (s, 1H, NC***H***Cq); 7.08- 7.18 (m, 2H, ***H_{aromat.}***); 7.28- 7.32 (t, 1H, ***H_{aromat.}***); 7.50- 7.59 (m, 2H, ***H_{aromat.}***); 7,78 (dt, 1H, ***H_{aromat.}***); 8.42 (d, 1H, ***H_{aromat.}***); 8.71 (d, 1H, ***H_{aromat.}***); 12.54 (s, O***H***)*.* **¹³C NMR** (CDCl₃, δ = ppm) 23.70 (NCH₂***C***H₂); 32.59 (NCH₂CH₂***C***H₂); 43.96 (CHCqOH); 46.76 (N***C***H₂); 52.12, 52.71 (COO***C***H₃); 59.64 (***C***HNCH₂); 60.50 (H₂CN***C***H); 97.10 (NCH***Cq***); 122.73 (***C6"***); 122.97 (***C4'***); 123.31 (***C6***') ; 124.02 (***C4"***); 136.59 (***C5"***); 137.74 (***C5***'); 148.33 (***C3"***); 148.75 *(C3');* 157.79 (***C1"***); 160.26 ***C1'***); 168.98 (***Cq***OH); 171.50 , 172.04 (***C***OOCH₃); 176.83 (***C***OOH). **IR** (cm⁻¹) 3030; 2980; 1710; 1620; 1430; 1350; 1210; 950; 845; 700. mp. 169°C.

**8-[(2R,6S)-3,5-bis(methoxycarbonyl)-4-oxo-2,6-di(pyridin-2-yl)piperidin-1-yl]octanoic acid (7)**: C₂₅H₂₉N₃O₇ (483.5 g/mol), yield: 43 %; **¹H NMR** (CDCl₃, δ = ppm, J = Hz) keto-enol ratio: 1:5, **¹H NMR** (CDCl₃, δ = ppm, *J* = *Hz)* 0.99- 1.09 (m, 2H, C***H***₂, enol); 1.16- 1.34 (m, 2H, C***H***₂, enol); 1.36- 1.45 (m, 2H, C***H₂***, enol); 2.05- 2.10 (m, 2H, ***CH₂,*** enol); 2.14- 2.21 (m, 2H, ***CH₂,*** enol); 2.39- 2.45 (m, 2H, NC***H***₂CH₂, enol); 3.58 (s, 6H, COOC***H₃***, enol); 3.64 (s, 6H, COOC***H₃***, ketone); 4.11 (d, 1H, J = 10.5, C***H***CqOH, enol); 4.27 (d, 2H, J = 6.8, C***H***C=O, ketone); 4.36 (d, 1H, CHNCH, J = 10.5, ketone, C***H***NCH, enol); 4.71 (d, 2H, J = 6.8, NCHCH, ketone); 4.89 (s, 1H, NC***H***Cq, enol); 7.20-7.35 (m, 2H, ***H6'*/*H6",*** enol, 2H, ***H4'*/*H4*",** enol, 2H, ***H4*'**, ketone); 7.48 (d, 2H, ***H6*'**, ketone); 7.67- 7.75 (m, 2H, ***H6'*/*H6",*** enol, 1H, ***H5'*/*H5",*** enol); 7.82-7.88 (m, 2H, H5', ketone, 2H, ***H5'*/*H5",*** enol); 8.42 (d, 1H, ***H3'*/*H3",*** enol); 8.51 (m, 2H, ***H3'*/*H3",*** ketone and enole). **¹³C NMR** (CDCl₃, δ = ppm) 24.0 (***C***H₂, enol); 25.6 (***C***H₂, ketone); 25.7 (***C***H₂, enol); 26.3 (***C***H₂, ketone); 26.8 (***C***H₂, ketone); 30.5 (***C***H₂, ketone); 30.7 (***C***H₂, enol); 33.6 (***C***H₂, enol); 46.0 (***C***H2, ***C5",*** enol); 51.8 (***C***H₂, ***C5"**,* ketone); 51.9 (COO***C***H₃, enol); 52.2 (COO***C***H₃, ketone); 56.6 (***C***HC=O, ketone); 59.5 (NCHCH, enol); 60.3 (N***C***HCq, enol); 63.3 (NCHCH, ketone); 98.0 (***Cq***=C-OH, enol), 122.2, 122.6, 122.7, 123.1 (***C3'*/** ***C3**"* and ***C5'*/** ***C**5"**,*** enol); 123.0, 123.4 (***C3*** and ***C5***, ketone); 136.6, 136.7 (***C4'*/ *C4",*** enol); 137.1 (***C4,*** ketone); 148.0, 148.2 (***C6'*/ *C6",*** enol); 148.5 (***C6*,** ketone); 157.4 (***C2***, ketone); 157.9, 161.6 (***C2'*/ *C2",*** enol); 166.0 (Cq=C-OH, enol); 169.0 (***C***OOCH₃, ketone); 170.6, 171.8 (***C***OOCH₃, enol); 174.4 (COOH); 198.6 (***C***=O). IR (cm⁻¹) 3013; 2940; 2858; 1737; 1254. mp. 130 °C.

**Dimethyl (2*R*,6*S*)-4-oxo-2,6-di(pyridine-2-yl)-1-(pyridine-2-yl-methyl)-piperidine-3,5-diearboxylate (8) :** C₂₅H₂₄N₄O₅ (460.5 g/mol), yield: 47%; **¹H NMR** (CDCl₃, δ = ppm, J = Hz) 3.51 (d, 1H, NC***H***₂); 3.53 (d, 1H, NC***H***₂); 3.61 (s, 6H, COOC***H***₃); 4.12 (d, 2H, ³Jₐₐ = 10.5, C***H***COOCH₃); 4.71 (d, 2H, ³Jₐₐ = 10.9, CHNCH); 7.17 (t, 2H, ***H5'*/*5"***); 7.26-7.31 (m, 2H, ***H6'*/*6"***); 7.66-8.10 (m, 2H, ***H3'*/*3"*** or ***H4'*/*4"***); 8.64 (t, 2H, ***H4'*/*4"*** or ***H3'*/*3"***). **¹³C NMR** (CDCl₃, δ = ppm) 53.6 (COO***C***H₃); 55.8 (***C***H₂); 62.9 (***C***HC=O); 67.9 (NCH); 123.7, 123.9 (***C2'*/*2"*/*C4'*/*4"***); 130.3 (***C5'*/*5"***); 136.3 (***C6'*/*6"***); 145.3 **(*C1'*/*1"***); 148.4 (***C3'*/*3"***); 170.1 (***C***OOCH₃); 192.4 (***C***=O). IR (cm⁻¹) 3020; 2980; 1710; 1640; 1500; 1405; 1310; 1119; 869; 727; 668. mp. 152-153 °C.

**Dimethyl (2R,6S)-1-benzyl-4-oxo-2,6-di(pyridin-2-yl)piperidine-3,5-dicarboxylate (9):** C₂₆H₂₅N₃O₅ (459.5 g/mol), yield: 65%; ¹H NMR (CDCl₃, δ = ppm, J = Hz) 3.53 (s, 2H, NC***H**₂*); 3.62 (s, 3H, COOC***H₃***); 3.68 (s, 3H, COOC***H₃***); 3.94 (d, 2H, C***H***COOCH₃); 4.72 (d, 2H, NC***H***); 6.69-6.75 (m, 2H, *CH_{Benzyl}*); 6. 95-6.99 (m, 3H, ***CH_{Benzyl}***)**;** 7.27 (t, 2H, ***H5'*/*5"***); 7.46-7.51 (m, 2H, ***H6'*/*6"***); 7.96-8.13 (m, 2H, ***H3'*/*3"*** or ***H4'*/*4"***); 8.36 (t, 2H, ***H4'*/*4"*** or ***H3'*/*3"***)**. ¹³C NMR** (CDCl₃, δ = ppm) 53. 2 (COO***C***H₃); 54.8 (***C***H₂); 64.6 (***C***HC=O); 69.7 (NCH); 122.7, 123.0 (***C2'*/*2"*/*C4'*/*4"***)**;** 127.4, 128.3, 128.5 (***C**H_{Benzyl}*); 134.3 (***C5'*/*5"***)**;** 135.4 (***C6'*/*6"***)**;** 138.6 (***Cq**_{Benzyl}*); 143.1 (***C1'*/*1"***); 147.2 (***C3'*/*3"***); 169.7 (***C***OOCH₃); 193.9 (***C***=O). **IR** (cm⁻¹) 2980; 1728; 1657; 1531; 1342; 1146; 836; 791; 726. mp. 154 °C.

**Dimethyl (2*R*,6*S*)-1-allyl-4-hydroxy-2,6-bis(4-nitrophenyl)-1,2,3,6-tetrahydropyridine-3,5- dicarboxylate (10):** C₂₄H₂₃N₃O₉ (497.5 g/mol); yield: 39 %; **¹H NMR** (CDCl₃, δ = ppm, J = *Hz*) 2.70-2.96 (NC***H***₂CH=CH₂); 3.54, 3.59 (2s, 3H, COOC***H***₃); 3.90 (dd, 2H, ³Jₐₐ = 9.9, ³J = 2.0, C***H***COOCH₃); 4.42 (d, 2H, J = 9.6, NC***H***₂CH=CH₂); 4.86-4.95 (m, 2H, NC***H***Cq, ***H_{B}***); 5.23 (dd, 1H, ³J_{AC} = 10.2, ²J_{AB} = 1. 4, ***H_{A}***); 5.71-5.86 (m, 1H, ***H_{C}***); 7.53-7.59 (m, 4H, ***H3'*/*3", H5'*/*H5"***)**;** 8.15-8.24 (m, 4H, ***H6'*/*6", H2'*/*H2"***)**;** 12.24 (O***H***). **¹³C NMR** (CDCl₃, δ = ppm) 51.5 (N***C***H₂CH=CH₂); 51. 9, 52.7 (COO***C***H₃); 55.3 (CHCOH) ; 59.7 (N***C***HCq); 62.6 (NCHCH) ; 101.4 (***Cq***=COH); 121.1 (NCH₂CH=***C***H₂); 123.4, 124.2 (***C6'*/*6", C2'*/*2"***)**;** 129.6, 130.1 (***C5'*/*5", C3'*/*3"***)**;** 130.2 (NCH₂***C***H=CH₂); 145.9, 151.0 (***C4'*/*4"***); 147.4, 148.2, (***C1'*/*1"***); 165.6 (Cq=COH); 168.6, 170.5 (***C***=O). IR (cm⁻¹) 2953; 1733; 1665; 1518; 1439; 1346; 1243; 696. mp.154-155 °C.

**Diethyl (2*R*,6*S*)-1-allyl-4-hydroxy-2,6-bis(4-nitrophenyl)-1,2,3,6-tetrahydropyridine-3,5-dicarboxylate (11) :** C₂₆H₂₇N₃O₉, (525.5 g/mol) yield: 28 %; **¹H NMR** (CDCl₃, δ = ppm, J = Hz) 1.00-1.07 (m, 6H, COOCH₂C***H***₃); 2.81-2.98 (m, 2H, NC***H***₂); 3.95-4.07 (m, 5H, COOC***H***₂CH₃, CHC-OH); 4.44 (d, 2H, ³Jₐₐ = 9.9, NCHCH); 4.86-4.95 (m, 2H; NC***H***Cq, ***H_{B}***); 5.25 (dd, 1H, ³J_{AC} = 12.2, ²J_{AB} = 0 . 9, *H_{A}*); 5.75-5.88 (m, 1H, ***H_{C}***); 7.57-7.67 (m, 4H, ***H3'*/*3", H5'*/*5"***); 8.17-8,25 (m, 4H, ***H2'*/*2", H6'*/*6"***); 12.38 (O***H***). **¹³C NBIR** (CDCl₃, δ = ppm) 14.0, 14.1 (COOCH₂***C***H₃); 51.4 (N***C***H₂); 55.1 (NCHCH); 61.4, 61.8 (COO***C***H₂CH₃); 62.2, 63.0 (NCH); 101.0 (C=COH); 123.3, 124.1 (***C2'*/*2", C6'*/*6"***); 123.9 (NCH***C***H=CH₂); 129.8 (NCHCH=***C***H₂); 130.0, 130.5 (***C3'*/*3", C5'*/*5"***)**;** 147.5 (***C4'*/*4"***); 148.2 (***C1'*/*1"***); 165.9 (C=COH); 168.1, 170.1 (***C***=O). **IR** (cm⁻¹) 2981; 2863; 1736; 1661; 1518; 1344; 1245; 700. mp.150-151 °C.

**Dimethyl (2*R*,6*S*)-1-benzyl-4-hydroxy-2,6-bis(4-nitrophenyl)-1,2,3,6-tetrahydropyridine-3,5-dicarboxylate (12):**
C₂₈H₂₅N₃O₉ (547.6 g/mol), yield: 12 %; **¹H NMR** (CDCl₃, δ = ppm, J = Hz) 3.52, 3.66 (2d, each 1H, *N*C***H₂***); 3.55, 3.59 (2s, each 3H, COOC***H₃***); 3.95 (dd, 1H, ³Jₐₐ = 9. 6, ³J = 1.8, NCHC***H***); 4.49 (d, 1H, ³Jₐₐ = 9.6, NCHCH); 4.79 (d, 1H, ⁴J = 1.5, NC***H***Cq); 6.78-6.83 (m, 2H, *C**H**_{Benzyl}*); 7.05-7.15 (m, 3H, *C**H**_{Benzyl}*); 7.42-7.52 (m, 4H, ***H3'*/*H3", H5'*/*H5"***); 8.06-8.11 (m, 4H, ***H2'*/*H2", H6'*/*H6"***); 12.27 (s, 1H, O***H***). **¹³C NMR** (CDCl₃, δ = ppm) 52.0; 52.7 (COO***C***H₃); 54.5 (NCHCH); 56.5 (N***C***H₂); 61.6 (NCHCH); 64.7 (NCHCq); 101.3 (***C***=COH); 123.2, 123.8 (***C2'*/*C2", C6'*/*C6"***); 127.5, 128.4, 129.0 (***C***H_{Benzyl}); 129.7, 130.0 (***C3'lC3", C5'*/*C5"***)**;** 137.0 (***Cq**_{Benzyl}*); 146.4 (***C4'*/*C4"***); 151.3 (***C1'*/*C1"***); 165.8 (C=***C***OH); 168.5, 170.4 **(*C***=O). IR (cm⁻¹) 2833; 1740; 1657; 1516; 1444; 1344; 1253; 731; 695. mp. 163-171 °C.

Diethyl **(2*R*,6*S*)-1-benzyl-4-hydroxy-2,6-bis(4-nitrophenyl)-1,2,3,6-tetrahydropyridine-3,5-dicarboxylate** (13) : C₃₀H₂₉N₃O₉ (575.7 g/mol), yield: 7 %; **¹H NMR** (CDCl₃, δ = ppm, J = Hz) 1.10-1.11 (m, 6H, COOCH₂C***H***₃); 3.49-3.72 (2d, each 1H, ²*J* = *14. 9,* NC***H₂***); 3.95-4.10 (m, 5H, COOC***H***₂CH₃, NCHCH); 4.48 (d, 1H, ³Jₐₐ = 9.6, NCHCH); 4.86 (d, 1H, ⁴J = 1.5, NC***H***Cq); 6.74-6.80 (m, 2H, *C**H_{Benzyl}***); 7.02-7.13 (m, 3H, C***H_{Benzyl}***); 7.43-7.54 (m, 4H, ***H3'*/*3", H5'*/*5"***); 8.04-8.12 (m, 4H, ***H2'*/*2", H6'*/*6"***); 12.39 (O***H***). **¹³C NMR** (CDCl₃, δ = ppm) 14.0, 14.1 (CH₂***C***H₃); 54.7 (CHCOH) ; 56.5 (N***C***H_{2 Benzyl}); 61.4, 61.8 (***C***H₂CH₃); 62.2 (NCHCH) ; 65.1 (N***C***HCq); 101.2 (***C***=COH); 123.2, 123.7 (***C2'*/*2", C6'*/*6"***); 127.4, 128.3, 128.8 (***C***H_{Benzyl}); 130.0, 130.3 **(*C3'*/*3", C5'*/*5"***)**;** 137.0 (***Cq***_{Benzyl}); 146.1, 151.2 (***C1'*/*1"***); 147.1, 148.0 (***C4'*/*4"***); 166.1 (C=***C***OH); 168.0, 170.1 (***C***=O). **IR** (cm⁻¹) 2988; 2857; 1740; 1655; 1516; 1345; 1243; 749; 698. mp. 181-184 °C.

**Dimethyl (2*R*,6*S*)-1-(4-chlorobenzyl)-2,6-bis(3-nitrophenyl)-4-oxopiperidine-3,5-dicarboxylate (14)** : C₂₈H₂₄N₃O₉Cl (582.0 g/mol), yield: 41 %; **¹H NMR** (CDCl₃, δ = ppm, J = Hz) 3. 47 (s, 2H, NC***H₂***); 3.55 (s, 3H, COOC***H₃***); 3.58 (s, 3H, COOC***H₃***); 4.02 (d, 2H, ³Jₐₐ = 10.8, C***H***COOCH₃); 4.59 (d, 2H, ³Jₐₐ = 10.8, NCH); 6.55-6.74 (m, 4H, *C**H_{Benzyl}***); 7.49 (t, 2H, J = 7. 3, ***H5'*/*5"***); 7.78-7.82 (m, 2H, ***H6'*/*6"***); 8.09- 8.13 (m, 2H, ***H9'*/*4"***)**;** 8.35 (t, 2H, ***H2'*/*2"***)**. ¹³C NMR** (CDCl₃, δ = ppm) 52.7 (COO***C***H₃); 55.4 (N***C***H₂); 63.9 (***C***HC=O); 68.1 (NCH); 113.9, 123.7, 123.9, 128.8, 129.9, 133.2, (***C2'*/*2", C4'*/*4", C5'*/*5", C6'*/*****6", C**H**_{Benzyl}***); 139.3 (***Cq_{Benzyl}***Cl); 140.8, 141.1, 148.3, 157.8 (***Cqₐᵣ***); 166.6 (***C***OOCH₃); 195.8 (***C***=O). **IR** (cm⁻¹) 3025; 2980; 1738; 1650; 1518; 1420; 1347; 1239; 880; 745; 697. mp. 144-146 °C.

**Dimethyl (2*R*,6*S*)-1-(4-methoxybenzyl)-2,6-bis(3-nitrophenyl)-4-oxopiperidine-3,5-dicarboxylate (15) :** C₂₉H₂₇N₃O₁₀ (577.5 g/mol), yield: 29 %; **¹H NMR** (CDCl₃, δ = ppm, J = Hz) 3.49 (s, 2H, NC***H₂***); 3.56 (s, 6H, COOC***H₃***); 3.71 (s, 3H, OC***H***₃); 4.01 (d, 2H, ³Jₐₐ = 11.1, C***H***C=O); 4.56 (d, 2H, ³Jₐₐ = 11.1, NCH); 6.57-6.62 (m, 4H, *C**H_{Benzyl}***); 7.52 (t, 2H, J = 7.6, ***H5'*/*5"***)**;** 7.84 (m, 2H, ***H6'*/*6"***); 8.12 (m, 2H, ***H2'*/*2"*** or ***H4'*/*4"***); 8.36 (s, 2H, ***H4'*/*4"***or ***H2'*/*2"***)**. ¹³C NMR** (CDCl₃, δ = ppm) 52.6 (COO***C***H₃); 54.4 (N***C***H₂); 55.4 (O***C***H₃); 63.9 (***C***HC=O); 67.5 (NCH); 113.8, 123.8, 123.9 129.8, 129.9, 135.0, (***C2'*/*2", C4'*/*4", C5'*/*5", C6'*/*****6", C**H**_{Benzyl}***); 137.3 (***Cq_{Benzyl}***CH₃); 141.1, 141.4, 148.5, 158.8 (***Cqₐᵣ***); 166.6 (***C***OOCH₃); 195.6 (***C***=O). **IR** (cm⁻¹) 3536; 3088; 2957; 1732; 1524; 1436; 1352; 1248; 1172; 813; 741; 694. mp. 159-161 °C.

**Dimethyl (2*R*,6*S*)-1-(4-methylbenzyl)-2,6-bis(3-nitrophenyl)-4-oxopiperidine 3,5-dicarboxylate (16):** C₂₉H₂₇N₃O₉ (561.6 g/mol), yield: 41 %; **¹H NMR** (CDCl₃, δ = ppm, J = Hz) 2.19 (s, 3H, CqC***H***₃); 3.52 (s, 2H, NC***H***₂); 3.56 (s, 6H, COOC***H***₃); 4.07 (d, 2H, ³Jₐₐ = 11.1, C***H***C=O); 4.57 (d, 2H, ³Jₐₐ = 11.1, NCH); 6.57 (d, 2H, J = 7.8, ***H3*/*5_{Benzyl}***); 6.84 (d, 2H, J = 7.8, ***H2*/*6_{Benzyl}***); 7.50 (t, 2H, J = 7.1, ***H5'*/*5"***); 7.87 (m, 2H, ***H6'*/*6"***); 8.08-8.15 (m, 2H, ***H2'*/*2"*** or ***H4'*/*4"***); 8.34 (s, 2H, ***H4'*/*4"*** or ***H2'*/*2"***)*.* **¹³C NMR** (CDCl₃, δ = ppm) 20.9 (***C***H₃); 52.7 (O***C***H₃); 55.0 (N***C***H₂); 63.8 (***C***HC=O); 67.9 (NCH) ; 123.9, 124.0 (***C2'*/*2"*/*C4'*/*4"***)*;* 128.4, 129.1 (***C***H***_{Benzyl}***); 129.9 (***C5'*/*5"***); 135.1 (CH₂***Cq_{Benzyl}***, ***C6'*/*6"***); 137.3 (***Cq_{Benzyl}***CH₃); 141.1 (***C1'*/*1"***); 148.5 (***C3'*/*3"***); 166. 6 (***C***OOCH₃); 195.5 (***C***=O). IR (cm⁻¹) 2956; 1732; 1530; 1437; 1350; 1259; 1171; 810; 736; 695. mp. 170-172 °C.

**Dimethyl (2*R*,6*S*)-1-benzyl-2,6-bis(3-nitrophenyl)-4-oxopiperidine-3,5-dicarboxylate (17):** C₂₈H₂₅N₃O₉ (547.5 g/mol), yield: 15 %; **¹H NMR** (CDCl₃, δ = ppm, J = Hz) 3.52 (s, 2H, NC***H₂***); 3.57 (s, 6H, COOC***H**₃*); 4.01 (d, 2H, ³Jₐₐ = 10.9, C***H***COOCH₃); 4.55 (d, 2H, ³Jₐₐ = 10.9, NCH); 6.65-6.72 (m, 2H, *C**H_{Benzyl}***)*;* 6.98-7.02 (m, 3H, C***H_{Benzyl}***); 7.47 (t, 2H, J = 8.0, ***H5'*/*5"***); 7.76-7.81 (m, 2H, ***H6'*/*6"***); 8.06-8.10 (m, 2H, ***H2'*/*2"*** or ***H4'*/*4"***); 8.34 (t, 2H, J = 1.9, ***H4'*/*4"*** or ***H2'*/*2"***). **¹³C NMR** (CDCl₃, δ = ppm) 52.6 (COO***C***H₃); 55.6 (***C***H₂); 63.9 (***C***HC=O); 68.3 (NCH); 123.8, 123.9 (***C2'*/*2"*/*C4'*/*4"***)*;* 127.1, 128.2, 128.3 (***C***H*_{Benzyl}*); 129.8 (***C5'*/*5"***); 135.1 (***C6'*/*6"***); 137.0 (***Cq**_{Benzyl}*); 141.3 (***C1'*/*1"***); 148.4 (***C3'*/*3"***); 166.6 (***C***OOCH₃); 195.7 (***C***=O). IR (cm⁻¹) 2953; 1748; 1528; 1441; 1348; 1242; 805; 741; 693. mp. 144-146 °C.

**Dimethyl (2*R*,6*S*)-1-allyl-4-oxo-2,6-diphenylpiperidine-3,5-dicarboxylate (18):** C₂₄H₂₅NO₅ (407.5 g/mol), yield: 81 %; keto-enol ratio: 4:3, **¹H NMR** (CDCl₃, δ = ppm, J = Hz), 2.79-2.86 (m, 4H, NC***H₂***CH=CH₂, ketone, NC***H₂***CH=CH₂, enol); 3.53 (s, 6H, COOC***H₃***, ketone); 3.64, 3.68 (2s, each 3H, COOC***H₃***, enol); 3.81-3.90 (m, 2H, C***H***C=O, ketone); 3.94 (d, 1H, ³Jₐₐ = 10.1, CHC-OH, enol); 4.35-4.45 (m, 3H, NCH, ketone, NCHCH, enol); 4.59-4.66 (m, 1H, NCH₂CH=C***H₂***, ketone); 4.84 (s, 1H, NC***H***Cq, enol); 5.06-5.19 (m, 3H, NCH₂CH=C***H₂***, ketone, NCH₂CH=C***H₂***, enol) ; 5. 67-5.87 (m, 2H, NCH₂C***H***=CH₂, ketone, NCH₂C***H***=CH₂, enol); 7.20-7.49 (m, 20H, C***H**ₐᵣ*, ketone, C***H**ₐᵣ*, enol) ; 12.45 (O***H***, enol). **¹³C NMR** (CDCl₃, δ = ppm) 46.8 (CHC-OH, enol); 49.6, 50.9 (N***C***H₂CH=CH₂, ketone and enol); 52.0, 52.7 (COO***C***H₃, enol); 52.2 (COO***C***H₃, ketone); 57.8, 57.9 (NCH, ketone); 64.9, 66.3 (NCH, enol); 99.0 (***C***=C-OH, enol); 117.7 (NCH₂CH=***C***H₂, ketone); 120.5 (NCH₂CH=***C***H₂, enol) ; 127.2, 127.8, 128.1, 128.2, 128.4, 128.5, 128.5, 128.9, 129.2, 130.1 (NCH₂***C***H=CH₂, enol and ***C***Hₐᵣ, ketone and enol); 136.8 (NCH₂***C***H=CH₂, ketone); 138.8, 139.5, 141,8 (***Cqₐᵣ**,* ketone and enol); 167.4 (C=***C̅***-OH, enol and ***C***OOCH₃, ketone); 171.1, 172.3 (***C***=O, enol); 197.5 (***C***=O, ketone). **IR** (cm⁻¹) 2951; 2847; 1738; 1653; 1438; 1273; 1209; 764; 698. mp. 142 °C.

**General procedure for the synthesis of the 3-methyl 2,6-di(pyridine-2-yl)-4-piperidone-3-monocarboxylates 19-24, modified after**^{30,31}
20 mmol of 2,4,6-trioxotetrahydropyrane were stirred in 30 mL methanol till dissolution and afterwards cooled to -20 °C. 20 mmol of the corresponding amine and 40 mmol pyridine-2-carboxaldehyde dissolved in 20 mL methanol, respectively, were added to the solution in parallel in a way that the reaction temperature does not exceed - 20 °C. After stirring for 2 h at -10 °C the solvent was removed *in vacuo* at room temperature. The obtained residue was covered with a small amount of methanol and the product crystallized at 4 °C. The analytical and spectroscopic data of **19-24** are in accordance with the ref. ^{30,31,32,33}

**Methyl (2'R,6'S)-1'-benzyl-4'-hydroxy-1',2',5',6'-tetrahydro-2,2';6',2''-terpyridine-3'-carboxylate (19):** C₂₄H₂₃N₃O₃ (401.5 g/mol), yield: 33% (53%)³¹, mp. 155 °C (163-164).³³

**Methyl (2'R,6'S)-4'-hydroxy-1'-(4-methylbenzyl)-1',2',5',6'-tetrahydro-2,2';6',2''-terpyridine-3'-carboxylate (20):** C₂₅H₂₅N₃O₃ (415.5 g/mol), yield: 72% (41%)³¹, mp. 145 °C (144).³³

**Methyl (2'R,6'S)-1'-(4-chlorobenzyl)-4'-hydroxy-1',2',5',6'-tetrahydro-2,2';6',2''-terpyridine-3'-carboxylate (21):** C₂₄H₂₂ClN₃O₃ (435.9 g/mol), yield 68% (49%)³⁴, mp. 141 °C (142).³³

**Methyl (2'R,6'S)-4'-hydroxy-1'-(4-methoxybenzyl)-1',2',5',6'-tetrahydro-2,2';6',2''-terpyridine-3'-carboxylate (22):** C₂₅H₂₅N₃O₄ (431.5 g/mol), yield: 43% (46%)³⁴, mp. 140 °C (140).³³

**Methyl (2'R,6'S)-4'-hydroxy-1'-(3-methoxybenzyl)-1',2',5',6'-tetrahydro-2,2';6',2''-terpyridine-3'-carboxylate (23):** C₂₅H₂₅N₃O₄ (431.5 g/mol), yield: 56% (63%)³⁴, mp. 143 °C (144 ^{o}_{C)} 33

**Methyl (2'R,6'S)-1'-allyl-4'-hydroxy-1',2',5',6'-tetrahydro-2,2';6',2''-terpyridine-3'-carboxylate (24):** C₂₀H₂₁N₃O₃ (351.4 g/mol), yield: 44%, mp. 158°C.³³

**Dimethyl (2*R*,6*S*)-4,4-dihydroxy-2,6-dimethyl-piperidinium-3,5-dicarboxylate hydrobromide (30a):** C₁₁H₂₀NO₆Br (342.2 g/mol), yield: 62 %.

**Dimethyl (2*RS*,6*RS*)-4-hydroxy-2,6-diethyl-piperidine-3,5-dicarboxylate (30b):** C₁₃H₂₁NO₅ (271.3 g/mol), yield: 97 % (oil).

**Dimethyl (2*RS*,6*RS*)-4-hydroxy-2,6-dipropyl-piperidinium-3,5-dicarboxylate hydrobromide (30c)**: C₁₅H₂₆NO₅Br (380.3 g/mol), yield: 50 %.

**Dimethyl (2*RS*,6*RS*)-4-hydroxy -2,6-dibutyl-3,5-piperidinium-dicarboxylate hydrobromide (30d)**: C₁₇H₃₀NO₅Br (408.3 g/mol), yield: 24 %.

**(2R,6S)-2,6-Dimethyl-4-oxopiperidinium chloride (31a):** C₇H₁₂NOCl (163.7 g/mol), yield: 95 %.

**(2*R*,6*S*)/(2*RS*,6*RS*)-2,6-Diethyl-4-oxopiperidinium chloride (31b):** C₉H₁₈NOCl (191.7 g/mol), yield: 93 %.

**(2*R*,6*S*)-/ (2RS,6RS)-2,6-Dipropyl-4-oxopiperidinium chloride** (31c): C₁₁H₂₂NOCl (219.8 g/mol), yield: 92 %.

**(2*R*,6*S*)-/ (2RS,6RS)-2,6-Dibutyl-4-oxopiperidinium chloride (31d):** C₁₃H₂₆NOCl (247.9 g/mol), yield: 90 %.

### Spiropiperidines (33-47):

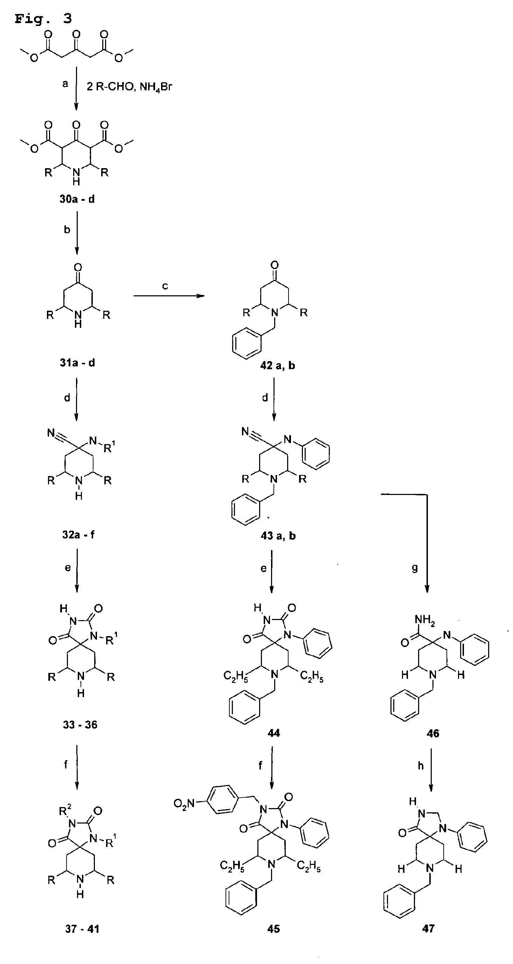

Spiropiperidine compounds according to Table 1, c (33-47) were synthesized according to conventional methods, as described in Fig. 3.

### 1-(3-Phenylpropyl)-4-piperidine oxime (48):

13.60 g (100.0 mmol) 4-oxo-piperidine monohydrate hydrochloride, 19.90 g (100.0 mmol) 1-bromo-3-phenylpropane and 69.10 g (500.0 mmol) potassium carbonate were mixed in 400 mL acetonitrile and stirred at 60 °C for 18 h. The solid was filtered off, washed with acetonitrile and the solvent was evaporated. The residue was diluted with 100 mL of dichloromethane and 50 mL sat. sodium hydrogencarbonate solution and the layers were separated. The aqueous layer was extracted twice with 50 mL dichloromethane and the combined organic layers were dried and evaporated. The pale yellow solid was used for the next step without further purification.

Potassium carbonate (10.84 g, 156.0 mmol) and hydroxylamine hydrochloride (21.60 g, 156.0 mmol) were dissolved with 200 mL abs. ethanol and a solution of 1-(3-phenylpropyl)-4-piperidinone (16.95 g 78.0 mmol) in 100 mL abs ethanol was added and refluxed for 1 h. The mixture was allowed to cool to 25 °C, filtrated and washed with ethanol. The solvent was evaporated and a yellow solid was achieved. Yield: 13.84 (59%). C₁₄H₂₀N₂O (232.3 g/mol); **¹H NMR** (d₄-MeOH, δ = ppm, J = Hz): 7.14 - 7.18 (5 H, m, Ph-H), 3.21 - 3.14 (4 H, m, Ph-C***H***₂-CH₂-C***H₂***-N), 2. 99 (2 H, t, J = 4.6, 2-H_{eq}, 6-H_{eq}), 2. 77 - 2.64 (2 H, m, 2-Hₐₓ, 6-Hₐₓ), 2. 60 - 2.49 (4 H, m, 3-H, 5-H), 2.03 - 1.95 (2 H, m, Ph-C***H***₂-CH₂-CH₂-N). **¹³C NMR** (d₄-MeOH, δ = ppm): 151.3 (-C=N), 141.6 (C-1 arom.), 129.7, 129.5 (C-2, C-2, C-2, C-6 arom.), 127.5 (C-4 arom.), 57.6 (Ph-CH₂-CH₂-***C***H₂-N), 53.7 and 52.5 (C-2, C-6, rotameres), 33.7 (Ph-***C***H₂-CH₂-CH₂-N), 29.3 (Ph-CH₂-***C***H₂-CH₂-N), 27.4 and 22.5 (C-3, C-5, rotameres), **IR** (ATR, cm⁻¹): 3147 (OH), 3074 (=C-H), 2946 (-CH₂), 1656 (-C=N), 1600 (-C=C- arom.), 954 (N-O), 763, 704 (out of plane). mp. 182.5 °C.

### 1-(2-Phenylpropyl)-4-oxo-piperidine O-(2,6-dichloro-benzyl)-oxime (49):

1.22 g (6.25 mmol) 2,6-dichlorobenzylchloride, 1.20 g (5.0 mmol) 1-(3-phenylpropyl)piperidin-4-oxime and 0.71 g (6.25 mmol) potassium-tert-butoxide were dissolved in dry 100 mL THF and stirred for 24 h at 55°C in the dark under Ar. The solvent was evaporated and the residue was diluted with 100 mL dichloromethane and 50 mL 2.0 M NaOH. The layers were separated and aqueous layer extracted with dichloromethane (2 x 80 mL). The combined organic layers were dried and evaporated to achieve a colorless solid. Yield: 0.68 g (28%). C₂₁H₂₄N₂OCl₂ (391.3 g/mol); **¹H NMR** (CDCl₃, δ = ppm): 7. 13 - 7.04 (8 H, m, aromatic); 5.23 (2 H, s, O-CH₂), 2.58 - 2.42 (8 H, m, 2-H, 3-H, 5-H, 6-H); 2.41 - 2.31 (4 H, m, Ph-C***H**₂*-CH₂-C***H**₂*-N); 1.82 - 1.77 (2 H, m, Ph-C***H₂***-CH₂-CH₂-N). **¹³C NMR** (CDCl₃, δ = ppm) : 152.1 (-C=N), 136.1 (C-1"), 133.4 (C-1'), 130.2 (C-2", C-6"), 128.5, 128.4, 128.2 (C-2', C-3' C-5', C-6', C-3", C-5''), 125.8, 125.6 (C-4', C-4''), 57.5 (CH₂-O), 53.4 (Ph-CH₂-CH₂-***C***H₂-N), 52.4 (Ph-***C***H₂-CH2-CH₂-N), 40.7 (C-2, C-6), 33.6 (C-3, C-5), 31.1 (Ph-***C***H₂-CH₂-CH₂-N). **IR** (ATR, cm⁻¹): 3026 (=C-H); 2945 and 2812 (-CH₂), 1660 (-C=N), 1563 (-C=C- arom.), 739, 705, 700, 642 (out of plane). mp. 147 °C.

### 4-(1,3-Dioxolan-2-yl)-N-(3-phenylpropyl)pyridinium bromide (50):

1.93 g (18.0 mmol) 4-pyridine-carbaldehyde, 2.26 g (36.0 mmol) ethylenglykole and 3.80 g (20.0 mmol) toluene-4-sulfonic acid were dissolved in 100 mL toluene and refluxed for 4 h to remove the water out of the reaction mixture. The solution was allowed to cool to 25 °C and the solvent was evaporated. The residue was diluted with 2.0 M NaOH (50 mL) and 50 mL dichloromethane. The layers were separated and the aqueous layer was extracted with dichloromethane (2 x 50 mL) and the combined organic layers were dried over anhydrous sodium sulfate, filtered and evaporated to achieve 4-(1,3-dioxolane-2yl)-pyridine as colorless oil, which was used in the next step without further purification. This 2.07 g (13.6 mmol) intermediate and 4.06 g (20.4 mmol) bromo-3-phenylpropane were dissolved in 20 mL toluene. The solution was filled into a sealed quartz glass vessel, equipped with a weflon plate (diameter 1.7 cm, thickness 0.3 cm) and was heated in the microwave oven at 110 °C for 2 h (5 min for 25 - 110 °C). After that time the solvent was evaporated and the oily residue was dissolved in 20 mL acetone and diethyl ether was added until the solution becomes turbid. The solution was cooled to -20 °C for 24 h. The crystals were filtered off, washed with diethyl ether and dried. Yield: 1.78 g (37 %) colorless crystals. C₁₇H₂₀NO₂Br (350.3 g/mol); **¹H NMR** (CDCl₃, δ = ppm): 9.45 (2 H, br, 2-H, 6-H pyridine), 7.94 (2 H, br, 3-H, 5-H pyridine), 7.21 - 7.06 (m, 5 H, Ph-H), 5.88 (s, 1H, O-CHO), 5.01 (br, 2 H, Ph-CH₂-CH₂-C***H***₂-N), 4.04 - 3.94 (m, 4 H, O-(CH₂)₂-O), 2.74 (t, 2 H, J = 6.6, Ph-C***H***₂-CH₂-CH₂-N), 2.36 - 2.29 (m, 2 H, Ph-C***H₂***-CH₂-CH₂-N⁺), **¹³C NMR** (CDCl₃, δ= ppm): 156.8 (pyridine C-4), 145.5 (pyridine C-2, C-6), 139.5 (phenyl C-1), 128.7 und 128.4 (phenyl C-2, C-3, C-5, C-6), 126.5 (pyridine C-3, C-5), 125.5 (phenyl C-4), 100.0 (O-CH-O), 65.9 (O-(CH₂)₂-O), 61.4 (Ph-CH₂-CH₂-***C***H₂-N), 33.1 (Ph-***C***H₂-CH₂-CH₂-N), 32.2 (Ph-CH₂-***C***H₂-CH₂-N). **IR** (ATR, cm⁻¹: 3026 (=C-H); 2976 and 2897 (-CH); 1708 (-C=C- aromatic); 1096 (C-O), mp. 78.1 °C.

### (1,3-Dioxolane-2-yl)-N-(3-phenylpropyl)piperidine hydrobromide (51)

1.84 g (8.18 mmol) 4-(1,3-dioxolane-2-yl)-1-(3-phenylpropyl)pyridinium bromide **50** and 40 mg PtO₂ were dissolved in 100 mL methanol and hydrogenated in a microwave hydrogenation reactor at 60°C and 20 bar for 1.5 h. The catalyst was filtered off and the solvent was evaporated. The residue was dissolved in acetone and crystallized with diethyl ether at 4°C the get a colorless solid. Yield 1.84 g (98%). C₁₇H₂₆O₂N₁Br (355.1 g/mol); **¹H NMR** (CDCl₃, δ = .ppm, J = Hz): 11.29 (1 H, br, NH⁺), 7.30 - 7.16 (5 H, m, arom.), 4.79 and 4.64 (1 H, d, rotameres, J = 5.6, O-CH-O), 4. 08 - 3.99 (4 H, m, O-CH₂-CH₂-O), 3.70 - 3.60 (2 H, m, Ph-CH₂-CH₂-C***H₂***-N), 3.07 - 2.71 (4 H, m, 2-H, 6-H), 2.68 - 2.16 (7 H, m, 3-H, 4-H, 5-H, Ph-C***H₂***-CH₂-CH₂-N), 1.76 - 1.69 (2 H, m, Ph-CH₂-C***H₂***-CH₂-N). **¹³C NMR** (CDCl₃, δ = ppm): 139.4 (phenyl C-1), 128.8, 128.4 (phenyl, C-2, C-3, C-5, C-6), 126.7 (phenyl C-4), 105.3 (O-CH-O), 66.0 (O-(CH₂)₂-O), 57.3 (Ph-CH₂-CH₂-***C***H₂-N), 52.6 (C-2, C-6), 38.7 (C-4), 32.9 (Ph-***C***H₂-CH₂-CH₂-N), 24.9 (Ph-CH₂-***C***H₂-***C***H₂-N), 23.9 (C-3, C-5). **IR** (ATR, cm⁻¹):3029 (=C-H), 2926 and 2885 (-CH₂), 1600 (-C=C- arom.), 1045 cm⁻¹ (C-O). mp. 132 °C (dec.)

### 1-1-(3-Phenyl-propyl)-piperidine-4-carbaldehyde oxime (52)

The acetal **51** (10.0 mmol, 3.55 g) was dissolved in 30 mL water and 0.2 mL sulphuric acid (98 %) were added. The mixture was heated up in the microwave within 4 min to 110 °C. The temperature was hold for 30 min and the solution was allowed to cool to 25 °C. 30 mL of 1.0 M Na₂HPO₄ and 6.65 g (100.0 mmol) hydroxylamine hydrochloride were added and the sodium hydroxide solution was added until a pH of 8.5 was achieved. The mixture was stirred at 25 °C for 18 h and the solution was alkalized with potassium carbonate and extracted with dichloromethane (3 x 100 mL). The combined organic layers were dried, filtered and evaporated. The oily residue was dissolved in diethyl ether, filtered and the filtrate was evaporated. Recrystallization from cyclohexane gave a colorless solid. Yield: 1.90 g (55 %), C₁₅H₂₂N₂O (246.4 g/mol); **¹H NMR** (CDCl₃, δ = ppm, J = Hz): 9.11 (1 H, br, OH), 7.64 - 7.10 (6 H, m, 5 H arom., HC=N), 2.91 - 2. 88 (2 H, m, 2-H_{eq}, 6-H_{eq}), 2.55 (2 H, t, J = 7.8, Ph-CH₂-CH₂-C***H₂***-N), 2.34 - 2.30 (2 H, m, 2-Hₐₓ, 6-Hₐₓ), 2.14 - 1.53 (9 H, m, 3-H, 4-H, 5-H, Ph-C***H₂***-CH₂-C***H₂***-N). **¹³C NMR** (CDCl_{3,} δ = ppm): 154.1 (HC=N), 141.9 (C-1, arom.), 128.4, 128.3 (C-2, C-3, C-5, C-6 arom.), 125.6 (C-4 arom.), 58.3 (C-2, C-6), 53.0, Ph-CH₂-CH₂-***C***H₂-N), 33.7 (C-4), 29.0 (C-3, C-5), 28.4, 28.2 (Ph-***C***H₂-***C***H₂-CH₂-N). **IR** (ATR, cm⁻¹): 3050 - 2750 (OH, br), 3021 (=CH), 2937, 2827 (-CH), 1652 (C=N), 1601, 1495 (C=C, arom.), 942 (N-O), 740 and 700 cm⁻¹ (out of plane), mp. 111 °C.

### 4-[(2,6-Dichloro-benzyloxyimino)-methyl]-1-(3-phenyl-propyl)-piperidinium hydrochloride (53)

The oxime **52** (0.7 g, 2.84 mmol) was dissolved in 40 mL abs. MeOH and potassium tert.-butoxide was added. The solution was stirred at 25 °C for 3 h and the solvent was removed *in vacuo.* The residue was suspended in 50 mL dry acetonitrile and 0.56 g (2.84 mmol) 2,6-dichlorobenzylchloride was added. The solution was refluxed for 2 h and the solvent was removed *in vacuo.* Column chromatography (silica gel, eluent: EtAc/EtOH 1:1 + 0.7 % triethylamine) gave a colorless oil. This oil dissolved in 10 mL diethyl ether and 1 mL 1.0 M hydrochloric acid in methanol was added. After 12 h at 4 °C a colorless solid was isolated. Yield: 0.51 g (40 %). C₂₂H₂₇Cl₃N₂O (412.5 g/mol); **¹H NMR** (d₆-DMSO, δ ppm, J = Hz): 10.28 (1 H, br, NH⁺), 7 . 50 - 7.18 (9 H, HC=N, 8 H arom.), 5.23 (2 H, s, CH₂-O), 3.58 - 3.38 (2 H, m, 2-H_{eq}, 6-H_{eq}), 3.00 - 2.80 (4 H, m, Ph-CH₂-CH₂-C***H₂***-N, 2-Hₐₓ, 6-Hₐₓ), 2.61 (2 H, t, J = 7.6, Ph-C***H₂***-CH₂-CH₂-N), 2.41 - 2.37 (1 H, m, 4-H), 2.00 - 1.68 (6 H, m, 3-H, 5-H, Ph-CH₂-C***H₂***-CH₂-N) . **¹³C NMR** (d₆-DMSO, δ=ppm) : 153.0 (C=N), 140.5 (C-1'), 136.1 (C-2'', C-6''), 132.0 (C-1''), 131.2 (C-4''), 128.6, 128.4, 128.2 (C-2', C-3', C-5', C-6', C-3'', C-5''), 126.1 (C-4'), 69.4 (CH₂-O), 55.6 (Ph-CH₂-CH₂-***C***H₂-N), 50.9 (C-2, C-6), 33.8 (C-4), 32.0 (Ph-***C***H₂-CH₂-CH₂-N), 26.0 (C-3, C-5), 24.8 (Ph-CH₂-***C***H₂-CH₂-N⁺). **IR** (ATR, cm⁻¹): 2910 (-CH), 2512 (NH⁺) 1715 (C=N), 1599 and 1563 (C=C, arom.), 1021 (C-O), 931 (N-O), 777, 767, 753 and 698 cm⁻¹ (out of plane), Mp.: 198 °C (dec).

### Trypanosome Assay according to^{34,35}

*Parasite culture:* Trypomastigote forms of *T. brucei brucei* laboratory strain TC 221 were cultured in Baltz medium according to standard conditions.³⁶

*In vitro cytotoxicity assays:* The test compounds were dissolved in DMSO or 0.1 M NaOH. A defined number of parasites (10⁴ trypanosomes per mL) were exposed in test chambers of 96-well plates to various concentrations of the test substances in a final volume of 200 µl. Positive (trypanosomes in culture medium) and negative controls (test substance without trypanosomes) were run with each plate.

The plates were then incubated at 37°C in an atmosphere of 5 % CO₂ for a total time period of 72 h. A reading was done at 48 h. The effect of test substances was quantified in ED₅₀ values by linear interpolation¹⁸ of three different measurements. The activity of the test substances was measured by light absorption in an MR 700 Microplate Reader at a wavelength of 550 nm with a reference wave length of 630 nm, using the Alamar Blue^{®}.

### Macrophage Assay according to³⁷

The macrophage cell line J774.1 was maintained in complete Click RPMI medium. For the experimental procedures cells were detached from the flasks with a rubber police, washed twice with PBS and suspended at 2 **x** 10⁶ cells mL⁻¹ in complete Click RPMI medium.

J774.1 macrophages were plated in 200 µl of complete RPMI medium without phenol red in 96-well plates in the absence or presence of various concentrations of the compounds and incubated for 24 h at 37 °C, 5 % CO₂, 95 % humidity. Following the addition of 20 µl of Alamar Blue, the plates were further incubated at similar conditions. The plates were then read 24 and 48 h later. Control experiments to examine the effect of cell density, incubation time and DMSO concentration were performed. Absorbance in the absence of compounds was set as 100 % of growth control.

### HIV-1 Infection Experiments:

HIV-1 infection experiments using the T-cell tropic (X4) strain NL4/3, the macrophage-tropic (R5) strain BaL were routinely performed using PM1 cells (virus laboratory strains and cells were obtained from the NIH AIDS Research and Reference Reagent Program). Cells were cultured in RPMI medium containing 10 % fetal calf serum (Pansystems GmbH) and antibiotics (penicillin and streptomycin). For HIV-1 infection, 5 x 10⁷ cells were resuspended in 500 µl culture medium without drugs and incubated at 37 °C for 3 h with 100 ng of HIV-1 viral stocks. After infection, cells were washed twice with PBS without Ca²⁺ and Mg²⁺ to avoid false positive p24 antigen determination. Cells were resuspended and identical aliquots (5 x 10⁵ / mL) of infected cells were further cultured in 24-well plates (triplicates) in the presence of the drugs (dissolved in DMSO) at various concentrations, or in medium with DMSO as control for the calculation of the inhibition of virus replication. Culture medium was changed and cells were splitted twice a week post-infection. Viability of the cells (as measured by Alamar Blue) and p24 antigen levels (as measured by ELISA; Innogenetics N.V.) were determined at different time points.

### Evaluation of the library in hematologic neoplasia, in particular in Chronic myeloid leukemia (CML) in vitro

Studies of differential protein expression in BCR-ABL positive leukemias have shown a primary overexpression of eIF-5A in CML cell lines *in vitro* and mononucleated cells of CML patients in vivo, which can be reversed under successful therapy of the disease. eIF-5A is the only known eukaryotic protein, for which, so far, a modification with Hypusine has been shown to be a mechanism for activation. This leads to a highly specific approach for a specific antileukemic therapy. In preliminary experiments, we could demonstrate a general activity of inhibitors of the modification with Hypusine (HI). Synergistic effects of inhibitors of tyrosins kinases and the compounds of the invention were observed.

### 1. Antiproliferative effect of inhibitors of Hypusine modification (HI).

Proliferation assays are carried out with human BCR-ABL positive (K562, BV173, EM-2 and others) and negative (HL60 and others) cells as well as with murine BaF3 cells, which, as wt-BaF3 are dependent on IL3, but proliferate independent from growth factors after expression of human BCR-ABL (BaF3-p210). Also, BaF3-p210 mutants with different resistance to the Tyrosine kinase (TK) inhibitor Imatinib (IM) may be used.

Several assays may be employed:
- Trypan Blue - proliferation is determined and proliferation curves are generated (typically after 24, 48 and 72 hours, as well as in a long time proliferation assay after 7 and 14 days);
- MTT Assay - IC₅₀ values are determined, so-called "dose-effect"-curves are generated. Synergistic effects of HIs and IM or other TKIs can be demonstrated.

### 2. Analysis of the cell cycle by staining with propidium iodide (PI)

This assay allows determination of the ploidy of DNA and quantification of the apoptotic fraction (sub G1 fraction) in mono- and combination therapy.

### 3. Biochemical effects of HIs on BCR-ABL dependent signal transduction pathways:

Changes in the phosphorylation status of P-CrkL, P-Stat5, P-c-Abl and P-Tyr may be detected, e.g., by Western blots or intracellular FACS.

### 4. Expansion assays with immunoaffinity purified CD34+ primary stem- and progenitor cells of healthy donors and patients in the different stages of CML, i.e. chronic phase (CP), accelerated phase (AP) and blast crisis (BC)

A Trypan Blue assay may be used. Typically, proliferation is determined and proliferation curves are generated after 3, 6, 9 and 12 days.

### Inhibition of HTLV

For the investigation wether the inhibition of DOHH by the compositions of the invention can influence replication of HTLV-I, the lymphoma cell line MT-2, which is latently infected by HTLV-I was used as a model system (Harada et al., 1985, Infection of HTLV-III/LAV in HTLV-I-carrying cells MT-2 and MT-4 and application in a plaque assay. Science 229, 563-566). MT-2 cells continually produce a high amount of virus particles which are secreted into the cell culture supernatant, and they grow quickly. The experimental approach is based on the comparison of the virus load of cells treated with the potential inhibitor and an untreated control. As the inhibitor was dissolved in DMSO, the same amount of this solvent was added to the control cells.

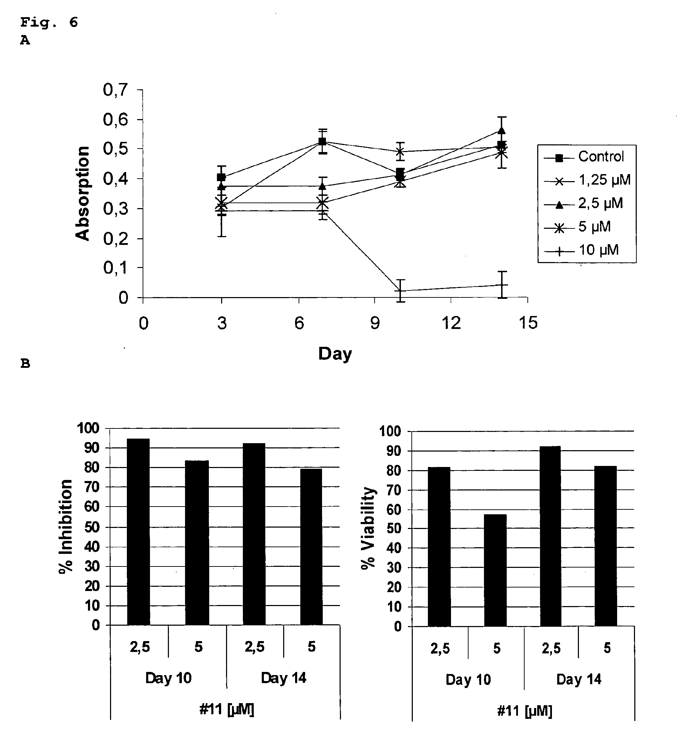

First, the subtoxic concentration of the inhibitor was determined by vitality assays. MT-2 cells were cultured for 14 days with 10, 5, 2.5 and 1.5 µM of the inhibitor as well as DMSO as a solvent control. In regular intervals, samples for determination of the vitality of the cells were taken. Vitality was tested with the alamarBlue^{®}-Assays. The results of inhibition with compound **11** are shown in Figure 6A.

After 10 days, a concentration of 10 µM of compound **11** led to death of 90 % of the cells, while in all other examined concentrations, cells death was comparable to the solvent control. Therefore, for further experiments, 2.5 and 5 µM of compound **11** was used.

For investigating the influence of inhibition of DOOH on the replication of HTLV-I, as before, MT-2 cells were cultivated with the determined subtoxic concentrations of compound **11** as well as a solvent control. In regular intervals, cell vitality was determined and the RNA of the virus particles from the supernatant was isolated. After reverse transcription with a primer specific for the gag region of HTLV-I RNA, the cDNA was quantified by real-time PCR. For detection of the amplified DNA, a gag-specific probe was used. A plasmid comprising the HTLV-I provirus served as a standard. Fig. 6B, left graph, shows the percentage of inhibition of virus production in relation to the solvent control. On day 10 and 14, the inhibition was over 90% for the cells treated with 2.5 µM of compound **11**, and over 80 % for the MT-2 cells treated with 5 µM. At the same time, vitality of the cells treated with 2.5 µM of compound **11** was higher than that of the cells treated with 5 µM of compound **11** (Figur 6B, right graph).

**Table 1:** Antimicrobial activity of all piperidone compounds (cf. Fig. 2, 3 and 4) against *T. brucei brucei* as well cytotoxicity measured in macrophages (J774.1), 7d. For comparison: Pentamidine diisothionate ED₅₀ (*T. brucei brucei*) *=* 0.0027 µM; Suramine ED₅₀ (*T. brucei brucei*) = 0.3 µM; Eflornitine ED₅₀ (*T. brucei brucei*) = 22.9 µM; ED₅₀ (*Trypanosoma b.b.*) > 100 µM; Ciclopiroxolamine ED₅₀ (*T. brucei brucei*) = 0.62 ± 0.22 µM, ED₅₀ (macrophages, 48 h) = 4.62 ± 0.97 µM; Amphotericin B IC₅₀ (J774.1 Macrophages) = 68.6 ± 16.48 µM
**a) 4-Oxo-piperidine 3,5-dicarboxylates**

| **N o** | **Ar** | **R¹** | **R²** | **ED₅₀ [µM] *T. brucei brucei*** | **ED₅₀ [µM] Macrophage after 48h** |
|---|---|---|---|---|---|
| **1** | 2-Pyridine | CH₃ | H | 28.09 ± 1.52 | > 100 |
| **2** | 2-Pyridine | CH₃ | Allyl | 26.48 ± 7.64 | > 100 |
| **3** | 2-Pyridine | CH₃ | 2-Hydroxyethyl | 22.84 ± 5.57 | 31.15 ± 0.14 |
| **4** | 2-Pyridine | CH₃ | 3-Hydroxypropyl | 18.29 ± 2.59 | 32.02 ± 0.63 |
| **5** | 2-Pyridine | CH₃ | 2-(2-Hydroxy-ethoxy)ethyl | 24.14 ± 4.27 | 38.48 ± 6.17 |
| **6** | 2-Pyridine | CH₃ | 3-Carboxypropyl | 19.29 ± 2.28 | 46.00 ± 5.92 |
| **7** | 2-Pyridine | CH₃ | 7-Carboxypentyl | 23.98 ± 4.48 | 33.83 ± 2.81 |
| **8** | 2-Pyridine | CH₃ | 2-Pyridinylmethyl | 4.62 ± 1.39 | 29.87 ± 2.32 |
| **9** | 2-Pyridine | CH₃ | Benzyl | 11.13 ± 1.91 | 33.99 ± 1.56 |
| **10** | 4-NO₂phenyl | CH₃ | Allyl | 2.41 ± 0.39 | 50.29 ± 11.70 |
| **11** | 4-NO₂phenyl | C₂H₅ | Allyl | 3.06 ± 0.55 | >100 |
| **12** | 4-NO₂phenyl | CH₃ | Benzyl | 3.01 ± 0.27 | 18.39 ± 2.93 |
| **13** | 4-NO₂phenyl | C₂H₅ | Benzyl | 2.72 ± 0.51 | >100 |
| **14** | 3-NO₂phenyl | CH₃ | 4-Chlorbenzyl | 10.57 ± 2.83 | 46.64 ± 9.86 |
| **15** | 3-NO₂phenyl | CH₃ | 4-Methoxybenzyl | 3.93 ± 1.10 | >100 |
| **16** | 3-NO₂phenyl | CH₃ | 4-Methylbenzyl | 2.66 ± 0.49 | 49.78 ± 16.80 |
| **17** | 3-NO₂phenyl | CH₃ | Benzyl | 2.49±0.79 | >100 |
| **18** | Phenyl | CH₃ | Allyl | 4.86 ± 0.63 | >100 |

**b ) 4-Oxo-piperidine 3-monocarboxylates**

| **N o** | **R** | **ED₅₀ [**µ**M] *T. brucei brucei*** | **ED₅₀ [**µ**M] macrophage after 48h** |
|---|---|---|---|
| **19** | Benzyl | 1.37 ± 0.02 | 33.17 ± 0.69 |
| **20** | 4-Methylbenzyl | 0.47 ± 0.17 | 34.87 ± 0.45 |
| **21** | 4-Chlorbenzyl | 0.32 ± 0.09 | 32.84 ± 0.49 |
| **22** | 4-Methoxybenzyl | 0.43 ± 0.17 | 29.86 ± 0.33 |
| **23** | 3-Methoxybenzyl | > 100 | > 100 |
| **24** | Allyl | 1.08 ± 0.48 | (35) |
| **4-Oxo-1,4,5,6-tetrahydropyridine 3-monocarboxylates** | | | |
| **25** | Benzyl | > 100 | n.d. |
| **26** | 4-Chlorbenzyl | n.d. | n.d. |
| **4-Oxo-1,4-dihydropyridine 3-monocarboxylates** | | | |
| **27** | 4-Chlorbenzyl | > 100 | > 100 |
| **28** | 4-Methylbenzyl | n.d. | n.d. |

**c) Spiropiperidines**

| **N o** | **R** | **R¹** | **R²** | **ED₅₀ [**µ**M] *T. brucei brucei*** | **ED₅₀ [µM] Macrophage after 48h** |
|---|---|---|---|---|---|
| **33** | CH₃ | Phenyl | | > 100 | >100 |
| **34** | C₂H₅ | Benzyl | | > 100 | >100 |
| **35** | C₃H₇ | Phenyl | | > 100 | 36.02 ± 3.97 |
| **36** | C₄H₉ | Phenyl | | 24.36 ± 14.10 | n.d. |
| **37** | CH₃ | Benzyl | 2-Hydroxyethyl | > 100 | > 100 |
| **38** | C₂H₅ | Phenyl | Benzyl | 20.28 ± 3.55 | n.d. |
| **39** | CH₃ | Benzyl | 4-Nitrobenzyl | 3.74 ± 0.77 | 42.44±14.49 |
| **40** | C₂H₅ | Phenyl | 2-Phenylethyl | 18.05 ± 5.34 | 32.30 ± 2.52 |
| **41** | C₂H₅ | Phenyl | 3-Phenylpropyl | 13.44 ± 6.69 | >100 |
| **44** | | | | 30.97 ± 2.18 | >100 |
| **45** | | | | 25.37 ± 1.61 | > 100 |
| **47** | | | | 44.65 ± 15.50 | n.d. |

**d) Oximes and corresponding ethers**

| | **ED₅₀ [**µ**M] *T. brucei brucei*** | **ED₅₀ [**µ**M] macrophage after 48h** |
|---|---|---|
| **48** | n.d. | n.d. |
| **49** | > 100 | >100 |
| **52** | > 100 | >100 |
| **53** | 3.86 ± 1.95 | 28.39±5.92 |

| | | |
|---|---|---|
| n.a. = no activity; n.d. not determined | | |

### Figure Legends:

- Fig. 1a:: Structural formulae of the DOHH inhibitors ciclopiroxolamine (1), L-mimosine (2), and 4-oxo-piperidone 3-carboxylate (3)
- Fig. 1b:: Points of variation of the piperidine skeleton
- Fig. 2:: Synthesis pathway to 4-oxo-piperidine 3,5-dicarboxylates **1 - 18 (**Reagents: (a) ArCHO, R²NH₂, -5 °C; (b) Ce(SO₄)₂, 0 - 5 °C), monocarboxylates **19 - 24,** and dihydropyridine and tetrahydropyridine compounds **25-28** (Reagents: (a) MeOH abs. ,25 °C; (b) 2 eq 2-pyridine aldehyde, R-NH₂, -5 °C; (c) Ce(SO₄)₂, 0 - 5 °C)
- Fig. 3:: Synthesis pathway to the spiro compounds **33-41, 44, 45**, and **47**. Reagents: (a) MeOH, EtOH, MeOH/H₂O or THF, 0 - 25 °C; (b) 37 % HCl, 70 °C; (c) R-Br, K₂CO₃, acetonitrile, 60 °C; (d) glacial acetic acid, aniline or benzylamine, TMSCN, 25°C; (e) 1. chlorsulfonylisocyanate, CHCl₃ abs., 25 °C; 2. 1 M HCl, reflux, 90 °C; (f) R²-Br, K₂CO₃, acetonitrile, reflux, 70 °C; (g) 1. H₂SO₄ conc., 25 °C; 2. NH₃ conc., 0 °C; (h) formamide, 200 °C

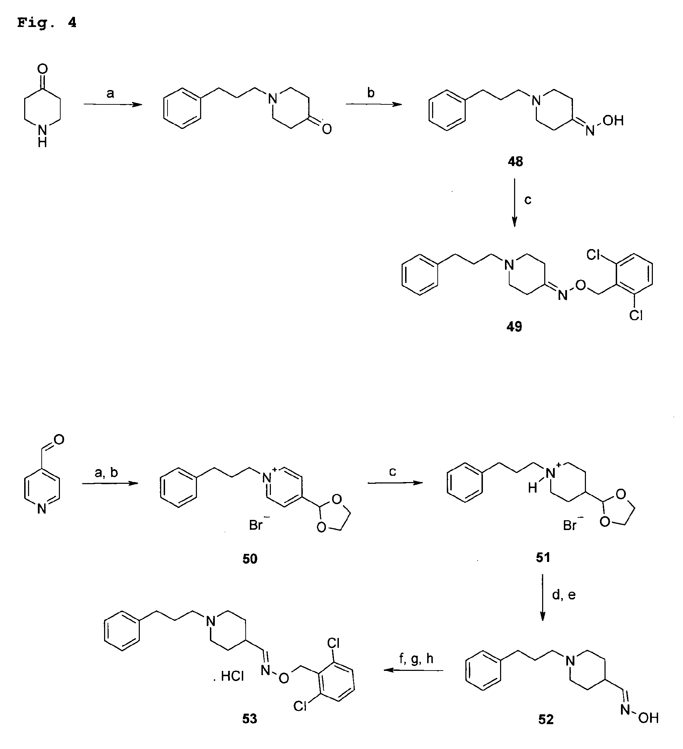

- Fig. 4:: Synthesis pathway to oximes **48** and **52** and their corresponding ethers **49** and **53**. Reagents and conditions for **48** and **49:** (a) Ph(CH₂)₃Br, K₂CO₃, CH₃CN, 60 °C; (b) K₂CO₃, NH₂OH. HCl, EtOH abs.; (c) 2,6- di-chlorobenzylchloride, KOtBu, 55 °C. Reagents and conditions for **52** and **53**: (a) ethylene glycole, pTosOH, toluene; (b) Ph(CH₂)₃Br, 2h, 90 °C, microwave; (c) PtO₂, H₂, 60 °C, 20 bar, microwave; (d) H₂SO₄, microwave; (e) NH₂OH- HCl; (f) KOtBu; (g) 2,6-dichlorobenzylchloride; (h) MeOH / HCl
- Fig. 5:: Inhibition of HIV-1 BaL replication by piperidones. HIV-1 BaL-infected PM1 cells were cultured in presence of the indicated concentrations of the compounds **11, 53** or DMSO as solvent control. Culture medium was changed at day 6 and 12 post-infection and the cells were split 1:1. p24^{Gag} antigen levels and cell viabilities were determined at day 6, 12 and 18. (A) The percentage of inhibition of virus replication in the drug-treated cell culture, as compared to the untreated controls is shown. (B) Cell viabilities from the same cultures were determined by alamarBlue^{™} assay (Serotec).
- Fig. 6A:: Vitality of MT-2 cells at different concentrations of compound **11** (cf. Table 1). MT-2 cells were cultivated for 14 days in different concentrations of **11** or a solvent control with DMSO. Vitality was determined in regular intervals with the alamarBlue^{®}-Assay.
- Fig. 6B:: Inhibition of HTLV-I virus production in MT-2 cells by **11**. MT-2 cells were cultivated for 14 days in medium with DMSO as a solvent control or 2.5 or 5 µM **11**. After 10 and 14 days, the vitality of the cells was controlled (right graph) and the cell-free supernatants were harvested. The virus particles obtained were pelleted by ultra centrifugation, viral RNA was isolated and then, a reverse transcription with primers specific for HTLV-I ***gag*** was carried out. The cDNA served as a template for the subsequent quantification by real-time PCR with a probe specific for HTLV-I ***gag.*** Percentage of inhibition versus vitality is shown with reference to the solvent control (left graph).
- Scheme 1:: Polyamine pathway and available inhibitors of the enzymes. DHS = Deoxyhypusine synthase; DOHH = Deoxyhypusine hydroxylase

### References

¹ Bevec, D.; Jaksche, H.; Oft, M.; Wohl, T.; Himmelspach, M.; Pacher, A.; Schebesta, M.; Koettnitz, K.; Dobrovnik, M.; Csonga, R.; Lottspeich, F.; Hauber, J. Inhibition of HIV-1 replication in lymphocytes by mutants of the Rev cofactor eIF-5A. Science 1996, 271, 1858-1860.
² Park, M.H.; Wolff, E.C., Folk, J.E. Hypusine: Its post-translational formation in eukaryotic initiation factor 5A and its potential in cellular regulation. Biofactors 1993, 4, 95-104.
³ Abbruzzese, A.; Park, M.H.; Folk, J.E. Deoxyhypusine hydroxylase from rat testis. Partial purification and characterization. J. Biol. Chem. 1988, 261, 3085-3089.
⁴ Park, M.H.; Wolff, E.C. Cell-free synthesis of deoxyhypusine. Separation of protein substrate and enzyme and identification of 1,3-diaminopropane as a product of spermidine cleavage. J. Biol. Chem. 1988, 263, 15264-15269.
⁵ Schäfer, B.; Hauber, I.; Bunk, A.; Heukeshoven, J.; Düsedau, A.; Bevec, D.; Hauber, J. Inhibition of multi-resistant HIV-1 by interference with cellular S-adenosylmethionine decarboxylase activity. J. Inf. Dis. 2006, 194, 740-750.
⁶ Hauber, I.; Bevec, D.; Heukeshoven, J.; Krätzer, F.; Horn, F.; Choidas A.; Harrer, T.; Hauber, J. Identification of cellular deoxyhypusine synthase as a novel target for antiretroviral therapy. J. Clin. Invest. 2005, 115, 76-85.
⁷ Hart, R.A.; Billaud, J.N.; Choi, S.J.; Phillips, T.R. Effects of 1,8-diaminooctane on the FIV Rev regulatory system. Virology 2002, 304, 97-104.
⁸ Andrus, L.; Szabo, P.; Grady, R. W.; Hanauske, A.-R.; Huima-Byron, T.; Slowinska, B.; Zagulska, S.; Hanauske-Abel, H. M. Antiretroviral effects of deoxyhypusyl hydroxylase inhibitors. Biochem. Pharmacol. 1998, 55, 1807-1818.
⁹ Müller, S.; Da'dara A.; Lürsen K.; Wrenger C.; Das Gupta, R.; Madhubala, R.; Walter, R. D. In the human malaria parasite Plasmodium falciparum, polyamines are synthesized by a bifunctional ornithine decarboxylase, S-adenosylmethionine decarboxylase. J. Biol. Chem. 2000, 275, 8097-8102.
¹⁰ Haider, N.; Eschbach, M. L; Dias Sde, S.; Gilberger, T. W.; Walter; R. D.; Luersen, K. The spermidine synthase of the malaria parasite Plasmodium falciparum: Molecular and biomchemical characterization of the polyamine synthesis enzyme. Mol. Biochem. Parasitol. 2005, 142, 224-236.
¹¹ Casero Jr, R.A.; Marton, L.J. Targeting polyamine metabolism and function in cancer and other hyperproliferative diseases. Nature Rev. Drug Discov. 2007, 7, 373-390
¹² Njuguna, J.T.; Nassar, M.; Hoerauf, A.; Kaiser, A.E. Cloning, expression and functional activity of deoxyhypusine synthase from Plasmodium vivax. BMC Microbiol. 2006, 6, 91
¹³ Clement, P.M.; Hanauske-Abel, H.M.; Wolff, E,C,; Kleinmann, H.K.; Park, M.H. The antifungal drug ciclopirox inhibits deoxyhypusine and proline hydroxylation, endothelial cell growth and angiogensis in vitro. Int. J. Cancer 2002, 100, 491-498.
¹⁴ Dong, Z.; Arnold, R.; Yang, Y.; Park, M.H.; Hrncirova, P.; Mechref, Y.; Novotny, M.V.; Zhang, J.T. Modulation of differentiation-related gene-1 expression by cell cycle blocker mimosine, revealed by proteomic analysis. Mol. Cell. Proteomics 2005, 4, 993- 1001.
¹⁵ Park, M.H. The post-translational synthesis of a polyamine-derived amino acid, hypusine, in the eukaryotic translation initiation factor 5A (eIF-5A). J. Biochem. 2006, 139, 1-9.
¹⁶ Cotton, F.A, Wilkinson, G. Advanced Inorganic Chemsitry, 5th Ed. Wiley, New York 1988, p.710
¹⁷ Haller, R. Metallchelate pyridyl-(2)-substituierter Piperidone und Piperidinole. Arch. Pharm. 1968, 301, 741-749.
¹⁸ Saeftel, M.; Sarite, R.S.; Njuguna, T.; Holzgrabe, U.; Ulmer, D.; Hoerauf, A.; Kaiser, A. Piperidones with activity against Plasmodium falciparum. Parasitol. Res. 2006, 99, 281-286.
¹⁹ Kaiser, A.; Ulmer, D.; Goebel, T.; Holzgrabe, U.; Saeftel, M.; Hoerauf, A. Inhibition of hypusine biosynthesis in plasmodium: a possible, new startegy in prevention and therapy of malaria. Mini-Rev. Med. Chem. 2006, 6, 1231-1241.
²⁰ http://www.who.int/tdr/diseases/tryp/;
   http://www.who.int/tdr/diseases/chagas/diseaseinfo.htm;
   http://www.who.int/leishmaniasis/en/
²¹ D'Silva, C. Human African trypanosomiasis: future propects for chemotherapy. Drugs Fut. 2007, 32, 149-160.
²² Merz, K.W.; Haller, R. Synthesen mit Pyridin- und Chinolinaldehyden. Pharm. Acta Helv. 1963, 38, 442-456.
²³ Ashauer-Holzgrabe, U.; Haller, R. Zur Umsetzung von N-benzylsubstituierten Piperidoncarbonsäuren mit Cer(IV)sulfat. Arch. Pharm. (Weinheim) 1986, 319, 1079-1083.
²⁴ Kuhl, U.; Englberger, W.; Haurand, M.; Holzgrabe, U. Diazabicyclo[3.3.1]nonanone-type ligands for the opioid receptors. Arch. Pharm. Pharm. Chem. 2000, 333, 226-230.
²⁵ Holzgrabe, U.; Piening, B.; Kohlmorgen, R.; Stoll, E. Synthese verschieden substituierter 5-Oxo-2,6-methano-2-benzazocine. Arch. Pharm. (Weinheim) 1988, 321, 917-920.
²⁶ Siener, T.; Holzgrabe, U.; Drosihn, S.; Brandt W. Conformational and configurational behaviour of κ-agonistic 3,7-diazabicyclo[3.3.1]nonan-9-ones - synthesis, nuclear magnetic resonance studies and semiempirical PM3 calculations. J. Chem. Soc. Perkin 2, 1999, 1827-1834.
²⁷ Smith, M.B.; March, J. March's advanced organic chemistry, 5th ed., Wiley-Interscience Publ., New York 2001, p. 1240.
²⁸ Röver, S.; Adam, G.; Cesura, A.M.; Galley, G.; Jenck, F.; Monsma, F.J.; Wichmann, J.; Dautzenberg, F.M. High-affinity, non-peptide agonists for the ORL1 (Orphanin FQ/Nociceptin) receptor. J. Med. Chem. 2000, 43, 1329-1338.
²⁹ Holzgrabe, U.; Erciyas, E. Synthese und Stereochemie potentiell stark analgetischer 2,4-m-diarylsubstituierter 3,7-Diazabicyclo[3.3.1]nonan-9-on-1,5-diester. Arch. Pharm. (Weinheim) 1992, 325, 657-663.
³⁰ Holzgrabe, U.; Piening, B.; Hesse, K.-F.; Höltje, H.-D.; Worch, M. Stereochemistry of 2,6-dipyridine substituted N-benzyl-4-piperidone mono- and dicarboxylates and the corresponding reduction products. Z. Naturforsch. 1988, 44b, 565-574.
³¹ Haller, R.; Kohlmorgen, R. Synthese und Struktur substituierter N-Benzyl-piperidoncarbonsäureester. Arch. Pharm. (Weinheim) 1976, 309, 206-214.
³² Haller, R. Zur Synthese 2,6-disubstituierter Piperidonmonocarbonsäureester. Arch. Pharm. 1965, 298, 787-794.
³³ Holzgrabe, U.; Piening, B.; Kohlmorgen, R.; Stoll, E. Cer(IV)oxidationen von β-Aminoketonen. 5. Mitt. Synthese verschieden substituierter 5-Oxo-2,6-methano-2-benzazocine. Arch. Pharm. (Weinheim) 1988, 321, 917-920.
³⁴ Räz, B.; Iten, M.; Grether-Bühler, Y.; Kaminsky, R.; Brun, R. The Alamar Blue assay to determine drug sensitivity of African trypanosomes (T. brucei rhodesiense and T. brucei gambiense) in vitro. Acta Trop. 1997, 68, 139-147.
³⁵ Huber, W.; Koella, J.C. A comparison of three methods of estimating EC50 in studies of drug resistance of malaria parasites. Acta Trop. 1993, 55, 257-261.
³⁶ Baltz, T.; Baltz, D.; Giroud, C.; Crocket, J. Cultivation in a semi-defined medium of animal infective forms of Trypanosoma brucei, T. equiperdum, T. evansi, T. rhodesiense and T. gambiense. EMBO J. 1985, 4, 1273-1277.
³⁷ Ahmed, S.A.; Gogal, R.M.; Walsh, J.E. A new rapid and simple non-radioactive assay to monitor and determine the proliferation of lymphocytes: an alternative to [3H]thymidine incorporation assay. J. Immunol. Methods 1994, 170, 211-224.

## Claims

1. A composition comprising a 4-oxo-piperidine-carboxylate according to Formula I 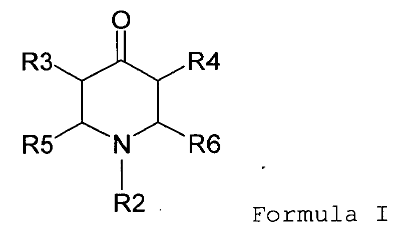 wherein R3 and R4 are selected from the group consisting of H, substituted or unsubstituted Alkyl and COOR1, wherein R1 is substituted or unsubstituted Alkyl, substituted or unsubstituted Alkylen or substituted or unsubstituted Aryl, with the proviso that at least one of R3 and R4 is COOR1,
R2 is selected from the group consisting of H, substituted or unsubstituted Alkyl, substituted or unsubstituted Alkylen or substituted or unsubstituted Aryl,
R5 and R6 are independently selected from the group consisting of substituted or unsubstituted Phenyl.

2. Composition of claim 1, wherein the substituted Phenyl is substituted or unsubstituted Nitrophenyl.

3. Composition of claims 1 and 2, wherein R5 and R6 are identical.

4. Composition of claims 2 or 3, wherein the Nitrophenyl is selected from the group comprising 4-NO₂-phenyl and 3-NO₂-phenyl.

5. Composition of claims 1-4, wherein R3 and R4 are COOR1.

6. Composition of claim 1-5, wherein R1 is Methyl, Ethyl, Propyl, Butyl, Isobutyl, Pentyl, Hexyl or Allyl.

7. Composition of claims 1-6, wherein R2 is selected from the group consisting of Allyl and substituted or unsubstituted Phenyl.

8. Composition of claim 7, wherein the substituted Phenyl is selected from the group comprising Benzyl, 4-Chlorbenzyl, 4-Methoxybenzyl and 4-Methylbenzyl.

9. Composition of claims 1-7, wherein R3 and R4 are COOR1 or H, R1 is Ethyl, R2 is Allyl and R5 and R6 are Nitrophenyl.

10. Composition of claim 9, wherein R5 and R6 are selected from the group comprising 4-NO₂-phenyl and 3-NO₂-phenyl.

11. Pharmaceutical composition comprising the composition of claims 1-10 and a pharmaceutically suitable excipient.

12. Use of a composition comprising a 4-oxo-piperidine-carboxylate according to Formula I 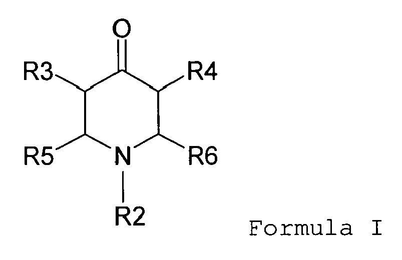 wherein R3 and R4 are selected from the group consisting of H, substituted or unsubstituted Alkyl, substituted or unsubstituted Alkylen or substituted or unsubstituted Aryl and COOR1, wherein R1 is substituted or unsubstituted Alkyl, substituted or unsubstituted Alkylen or substituted or unsubstituted Aryl, with the proviso that at least one of R3 and R4 is COOR1,
R2 is selected from the group consisting of H, substituted or unsubstituted Alkyl, substituted or unsubstituted Alkylen or substituted or unsubstituted Aryl,
R5 and R6 are independently selected from the group consisting of substituted or unsubstituted Aryl,
for the preparation of a pharmaceutical composition for treatment of a retroviral disease, a proliferative disease or trypanosomiasis.

13. Use of claim 12, wherein the composition is the composition of claims 1 to 10.

14. Use of claim 12, wherein R5 and/or R6 is Pyridine.

15. Use of claim 14, wherein R3 or R4 is H, R5 and R6 are 2-Pyridine and R2 is selected from the group comprising Benzyl, 4-Methylbenzyl, 4-Chlorbenzyl, 4-Methoxybenzyl and Allyl, and the composition is used for treatment of trypanosomiasis.

16. Use of claim 13, wherein R3 and R4 are COOR1, R1 is Ethyl or Methyl, R2 is selected from the group consisting of Allyl, Benzyl or 4-Methyoxybenzyl, R5 and R6 are selected from the group consisting of 4-NO₂-phenyl and 3-NO₂-phenyl and the composition is used for treatment of trypanosomiasis.

17. Use of claims 13, wherein R1 is Ethyl or Methyl, R2 is selected from the group consisting of Allyl and Benzyl, R5 and R6 are 4-NO₂-phenyl and the composition is used for treatment of a retroviral disease or proliferative diseases.

18. Use of claim 17, wherein R3 and R4 are COOR1 and R1 is Ethyl or Methyl, R2 is Allyl, and the composition is used for treatment of a retroviral disease.

19. Use of claims 12, 13, 17 or 18, wherein the retroviral disease is HTLV or HIV.

20. Use of claims 12, 13 or 17, wherein the proliferative disease is chronic myeloid leukemia.

21. Use of claims 12-16, wherein the trypanosomiasis is Chagas' disease or sleeping sickness.

22. Method for the preparation of the composition of claims 1 to 11, wherein an amine, pyridine-2-carboxaldehyde or a nitrobenzaldehyde are reacted with an oxoglutarate.

23. Method for identifying a composition having high activity against HIV, HTLV or trypanosomiasis or antiproliferative activity, and having a low toxicity, comprising synthesizing a composition of claims 1 - 10 and testing said composition for its activity and its toxicity, optionally comprising repeating synthesis with substituted or modified reactants and repeating testing.

24. Method for the preparation of a pharmaceutical composition for treatment of a retroviral disease, a proliferative disease or trypanosomiasis, comprising carrying out the method of claim 23, selecting a composition with high activity and low toxicity and formulating said composition with a pharmaceutically suitable excipient.
